(19) Europäisches Patentamt / European Patent Office / Office européen des brevets

(11) **EP 3 972 412 B1**

(12) **EUROPÄISCHE PATENTSCHRIFT**

(45) Veröffentlichungstag und Bekanntmachung des Hinweises auf die Patenterteilung:
**06.08.2025 Patentblatt 2025/32**

(21) Anmeldenummer: **20723891.6**

(22) Anmeldetag: **12.05.2020**

(51) Internationale Patentklassifikation (IPC):
***A01N 25/30** (2006.01)* ***A01N 63/22** (2020.01)*
***A01N 63/30** (2020.01)* ***A01N 63/38** (2020.01)*
***A61K 35/741** (2015.01)* ***A23L 33/10** (2016.01)*
***A23L 33/135** (2016.01)* ***A01P 3/00** (2006.01)*
***A01P 7/00** (2006.01)* ***A23K 10/18** (2016.01)*
***A23K 20/00** (2016.01)* ***A23L 29/10** (2016.01)*

(52) Gemeinsame Patentklassifikation (CPC):
(C-Sets verfügbar)
**A01N 25/30; A23K 10/18; A23K 20/00; A23L 29/10; A23L 33/10; A23L 33/135; A61K 35/741** (Forts.)

(86) Internationale Anmeldenummer:
**PCT/EP2020/063123**

(87) Internationale Veröffentlichungsnummer:
**WO 2020/234035 (26.11.2020 Gazette 2020/48)**

(54) **VERWENDUNG VON POLYGLYCERINESTERN ALS TRÄGER FÜR MIKROBIOLOGISCHE WIRKSTOFFE**

USE OF POLYGLYCEROL ESTERS AS CARRIERS FOR MICROBIOLOGICAL SUBSTANCES

UTILISATION D’ESTERS DE POLYGLYCÉRINE EN TANT QUE PORTEURS DE SUBSTANCES ACTIVES MICROBIOLOGIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **20.05.2019 EP 19175286**

(43) Veröffentlichungstag der Anmeldung:
**30.03.2022 Patentblatt 2022/13**

(73) Patentinhaber: **Evonik Operations GmbH**
**45128 Essen (DE)**

(72) Erfinder:
- **HÄNSEL, René**
  **46282 Dorsten (DE)**
- **SINGER, Alissa**
  **64331 Weiterstadt (DE)**
- **SKRABANIA, Katja**
  **45136 Essen (DE)**
- **KLEINEN, Jochen**
  **52525 Heinsberg (DE)**
- **BERGER, Tanja**
  **45359 Essen (DE)**
- **KLEINSCHMIDT, Kevin**
  **45884 Gelsenkirchen (DE)**

(74) Vertreter: **Evonik Patent Association**
**c/o Evonik Industries AG**
**IP Management**
**Postcode 84/339**
**Rodenbacher Chaussee 4**
**63457 Hanau (DE)**

(56) Entgegenhaltungen:
**WO-A1-2012/163322** **WO-A1-2016/050726**
**WO-A1-2016/055344** **WO-A1-2018/078067**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **.: "Spray Adjuvants Registered for Use on Aquatic Sites in Washington", 15 May 2017 (2017-05-15), Olympia, WA, U.S.A., pages 1 - 5, XP055611941, Retrieved from the Internet <URL:https://static1.squarespace.com/static/50d37c2ce4b09ff030bc2f7b/t/5ada2b7c575d1ffe151bf1ff/1524247421643/Spray+Adjuvants+Registered+For+Use+on+Aquatic+Sites+in+Washington+201705....pdf> [retrieved on 20190809]**
- **.: "BREAK-THRU SP 133 Product Label", 9 May 2017 (2017-05-09), Hopewell, VA, U.S.A., pages 1 - 2, XP055611940, Retrieved from the Internet <URL:http://cru66.cahe.wsu.edu/~picol/pdf/WA/63379.pdf> [retrieved on 20190809]**


Bemerkungen:

Die Akte enthält technische Angaben, die nach dem Eingang der Anmeldung eingereicht wurden und die nicht in dieser Patentschrift enthalten sind.

(52) Gemeinsame Patentklassifikation (CPC): (Forts.)

C-Sets

**A01N 25/30, A01N 63/22, A01N 63/30, A01N 63/38**

## Beschreibung

Die vorliegende Erfindung betrifft die Verwendung von Zusammensetzungen, die mindestens einen Polyglycerinester und vorzugsweise mindestens einen Emulgator umfassen, als Träger für mindestens einen mikrobiologischen Wirkstoff; Zusammensetzungen, die sowohl den Träger als auch den mikrobiologischen Wirkstoff umfassen; Verfahren zur Steigerung der Lagerstabilität des mikrobiologischen Wirkstoffs; sowie die Verwendung dieser Zusammensetzungen für die Behandlung von Pflanzen, für die Behandlung von Saatgut, für die Behandlung von Böden, als Biostimulanz, als probiotisches Nahrungsergänzungsmittel oder probiotisches Futtermitteladditiv.

Erfindungsgemäss ist der Emulgator ausgewählt aus der Gruppe bestehend aus Sorbitanfettsäureestern und ethoxylierten Sorbitanfettsäureestern. Erfindungsgemäss ist der mindestens eine mikrobiologische Wirkstoff ausgewählt aus der Gruppe bestehend aus Mikroorganismen, Organen von Mikroorganismen und ihren Mischungen.

In der Landwirtschaft werden Mikroorganismen für eine Vielzahl nützlicher Anwendungen eingesetzt, wie zum Beispiel für den biologischen Pflanzenschutz, zur biologischen Pflanzenstärkung oder zur biologischen Bodenverbesserung. Des Weiteren werden Zusammensetzungen, die lebende Mikroorganismen enthalten, auch für die Behandlung von Saatgut verwendet. Anwendungsgebiet ist also insbesondere die Land- und Forstwirtschaft einschließlich des Garten- und Obstbaus sowie der Kultur von Zierpflanzen und die Anlage und Pflege von Rasenflächen. Zudem kommen Zusammensetzungen, die lebende Mikroorganismen enthalten, auch als Probiotika in Nahrungs- und Futtermitteln oder als probiotische Arzneimittel zur Anwendung.

Biologische Pflanzenschutzmittel - auch als Biopestizide bezeichnet - werden zunehmend in der Landwirtschaft eingesetzt, da sie sie helfen, chemische Pestizide zu ersetzen oder ihren Einsatz zu verringern und so Rückstände chemischer Pestizide auf Lebensmitteln zu reduzieren. Bei Resistenzen von Pflanzenkrankheits- und Schaderregern gegenüber chemischen Pestiziden stellen biologische Pflanzenschutzmittel Alternativen dar. Der Einsatz von biologischen Pflanzenschutzmitteln wird durch die aktuelle Umweltgesetzgebung zunehmend gefördert, da sie sich natürlicher Regulationsmechanismen bedienen, welche sich im Laufe der Evolution entwickelt haben, und damit umweltschonend sind. Biologische Pflanzenschutzmittel finden beispielsweise Verwendung als Fungizide, Insektizide, Nematizide oder Herbizide und werden zur vorbeugenden Behandlung oder kurativen Bekämpfung von Pflanzenkrankheitserregern sowie Schaderregern eingesetzt. Biologische Wirkstoffe sind beispielsweise in The Manual of Biocontrol Agents, 2001, The British Crop Protection Council angegeben.

Als Pflanzenschutzmittel werden gemäß Artikel 2 (1) der VERORDNUNG (EG) Nr. 1107/2009 DES EUROPÄISCHEN PARLAMENTS UND DES RATES vom 21. Oktober 2009 über das Inverkehrbringen von Pflanzenschutzmitteln und zur Aufhebung der Richtlinien 79/117/EWG und 91/414/EWG des Rates Produkte in der dem Verwender gelieferten Form bezeichnet, die aus Wirkstoffen, Safenern oder Synergisten bestehen oder diese enthalten und für einen der nachstehenden Verwendungszwecke bestimmt sind:

a) Pflanzen oder Pflanzenerzeugnisse vor Schadorganismen zu schützen oder deren Einwirkung vorzubeugen, soweit es nicht als Hauptzweck dieser Produkte erachtet wird, eher hygienischen Zwecken als dem Schutz von Pflanzen oder Pflanzenerzeugnissen zu dienen;

b) in einer anderen Weise als Nährstoffe die Lebensvorgänge von Pflanzen zu beeinflussen (z. B. Wachstumsregler);

c) Pflanzenerzeugnisse zu konservieren, soweit diese Stoffe oder Produkte nicht besonderen Gemeinschaftsvorschriften über konservierende Stoffe unterliegen;

d) unerwünschte Pflanzen oder Pflanzenteile zu vernichten, mit Ausnahme von Algen, es sei denn, die Produkte werden auf dem Boden oder im Wasser zum Schutz von Pflanzen ausgebracht;

e) ein unerwünschtes Wachstum von Pflanzen zu hemmen oder einem solchen Wachstum vorzubeugen, mit Ausnahme von Algen, es sei denn, die Produkte werden auf dem Boden oder im Wasser zum Schutz von Pflanzen ausgebracht.

Im Rahmen der vorliegenden Erfindung wird vorzugsweise auf die o.g. Definition für den Begriff "Pflanzenschutzmittel" zurückgegriffen.

Nach der vorläufigen Definition des *European Biostimulants Industry Council* (EBIC) enthalten Biostimulanzien solche Substanzen und/oder Mikroorganismen, deren Funktion bei der Anwendung auf Pflanzen oder in der Rhizosphäre es ist, natürliche Prozesse zu stimulieren, um die Nährstoffaufnahme, Nährstoffeffizienz, Toleranz gegenüber abiotischem Stress und die Qualität der Kulturpflanzen/Ernteprodukte zu verbessern (http://www.biostimulants.eu/). Beispielsweise können die Mikroorganismen *Trichoderma* spp., *Pythium oligandrum, Bacillus* spp., *Pseudomonas* spp. und *Streptomyces* spp. Reaktionen in Pflanzen hervorzurufen, die zu einer erhöhten Widerstandsfähigkeit gegen Krankheitserreger oder andere Stressfaktoren, wie Trockenheit, schlechte Nährstoffversorgung, ungünstige pH-Werte und/oder hohe Salzgehalte im Boden, führen. Die Mikroorganismen *Trichoderma* spp., *Penicillium bilaii, Azotobacter* spp., *Azotomonas* spp., *Azospirillum* spp. und *Rhizobium* spp. können beispielsweise zu einer Verbesserung der Nährstoffverfügbarkeit im Boden oder direkt an der Pflanzenwurzel führen.

Einer breiten Verwendung mikrobiologischer Wirkstoffe für den biologischen Pflanzenschutz, zur biologischen Pflanzenstärkung oder zur biologischen Bodenverbesserung steht bislang ihre im Vergleich zu vielen chemischen Produkten geringere Wirksamkeit entgegen. Diese geringere Wirksamkeit beruht zum Beispiel auf einer unzureichenden Überlebensfähigkeit der Mikroorganismen in der Formulierung während der Lagerung. In der Applikation erreicht möglicherweise zu wenig Wirkstoff den Zielort auf der Pflanze oder im Boden, wo dieser gegebenenfalls durch Umwelteinflüsse rasch abgebaut wird. Diese nachteiligen Aspekte können jedoch durch einen geeigneten Träger verbessert werden.

Das biologische Pflanzenschutzmittel, welches auf Mikroorganismen als aktiven Bestandteil beruht, sowie die Biostimulanzien, werden üblicherweise in Form einer Formulierung vor der Anwendung in Wasser verdünnt. Diese Formulierungen können beispielsweise feste Formulierungen, wie wasserdispergierbare Pulver (WP, wettable powder) oder wasserdispergierbare Granulate (WG, water dispersible granules), sein, aber auch flüssige Formulierungen, wie Öldispersionen (OD, oil dispersions), Suspensionskonzentrate (SC, suspension concentrate) oder Dispersionskonzentrate (DC, dispersion concentrate).

Der Träger bringt die Mikroorganismen in eine handhabbare Form, sodass sie im Wasser verteilt und appliziert werden können. Da viele Mikroorganismen wie einige Gattungen von Pilzkonidien wasserabweisend sind, kommt dem Träger insbesondere die Aufgabe zu, diese im Wasser verträglich einzustellen. Außerdem soll die Formulierung auch die Überlebensfähigkeit der Mikroorganismen während Transport und Lagerung gewährleisten. Der Träger soll auch gewährleisten, dass die Anwendung mittels Sprühgeräten erfolgen kann. Die Aggregation der Mikroorganismen soll also vermieden werden, damit ein Verstopfen von Düsen ausgeschlossen werden kann. Die Träger soll dabei vorteilhafterweise auch solche Substanzen enthalten, die die Dispergierung und Verteilung der Mikroorganismen im Wasser gewährleisten, sowie die Aufbringung der Spritzbrühe auf den Pflanzen oder den Boden erleichtern.

In der Praxis werden Formulierungen chemischer und biologischer Pflanzenschutzmittel vor ihrer Anwendung vom Anwender in Wasser verdünnt. Dazu werden die Pflanzenschutzmittel üblicherweise einem Tank mit Wasser als Inhalt zugegeben und unter Rühren in der sogenannten Spritzbrühe verteilt. Diese Spritzbrühe ist eine anwendungsfertige Verdünnung der Pflanzenschutzmittel. Zur Bestellung von Agrarflächen werden diese Spritzbrühen über den zu behandelnden Pflanzen zerstäubt. Zerstäubung bedeutet in diesem Zusammenhang die Tröpfchenbildung durch mechanische Einwirkung auf ein flüssiges Medium, bevorzugt durch Rotation von Gegenständen und/oder durch Entspannung (Verminderung des Drucks) an kleinen Öffnungen. Besonders bevorzugt wird die Spritzbrühe in Form eines mit Hilfe von Düsen erzeugten Sprays aufgebracht. Für die Bestellung von Agrarflächen werden in der Regel 100 bis 1000 Liter, optimalerweise 100 bis 400 Liter Spritzbrühe pro Hektar versprüht. In Ausnahmefällen wird von diesen Grenzen aber abgewichen. Die Grenzen können also durchaus nach oben oder unten variieren. Soi werden in sogenannten *Low-Volume* Applikationen beispielsweise sehr geringe Mengen von bis zu 1,5 l/ha versprüht, während bei Applikation mit der sogenannten Lanzentechnik sehr hohe Mengen von bis zu 15000 l/ha erreicht werden können. Der Zerstäubungsvorgang kann hierbei entweder aus Höhenlagen, beispielsweise durch das Versprühen von Spritzbrühen aus einem Flugzeug, oder aus erdbodennahen Lagen, beispielsweise durch Versprühen von Spritzbrühen mittels eines an einem Traktor befestigten Spritzgestänges, erfolgen. Andere Geräte, wie Sprühlanzen, oder Rückenspritzen sind ebenfalls bekannt für die Ausbringung von Spritzbrühen. Die Spritzbrühe wird also üblicherweise mittels einer Düse auf die Pflanzen oder den Boden in einer vorgegebenen Dosierung gesprüht. Die Sprühtropfen sollen sich dabei gut auf der Pflanze oder dem Boden verteilen, damit eine optimale Wirkung gewährleistet ist.

Zur Verbesserung der biologischen Wirksamkeit (auch als Effektivität bezeichnet) chemischer Pflanzenschutzmittel ist es gängige Praxis sogenannte "Adjuvantien", auch als "Zusatzstoffe" oder "Adjuvants" bezeichnet, einzusetzen. Adjuvantien werden dabei üblicherweise der wässrigen Spritzbrühe kurz vor dem Ausbringen und Aufsprühen als Tankmischungsadditiv zugesetzt oder direkt in Pflanzenschutzmittelformulierungen integriert. Die Adjuvantien werden üblicherweise in Konzentrationen von 0,001 Vol.-% bis 1 Vol.-% der Spritzbrühe zugesetzt. Die Adjuvantien reduzieren die Oberflächenspannung von Wasser und sorgen für ein verbessertes Anhaften sowie Benetzen der Sprühtropfen an den hydrophoben Blättern der Pflanze und somit für eine großflächige und homogene Verteilung des Pflanzenschutzmittels. Auch verbessern sie das Eindringen und die Verteilung der aktiven Bestandteile der Spritzbrühe in den Boden. Dadurch wird die biologische Wirksamkeit erhöht. Ebenso können Adjuvantien auch die Wirksamkeit mikrobiologischer Pflanzenschutzmittel verbessern und in Abhängigkeit von der Art der Formulierung als Dispergiermittel, Emulgator und Netzmittel eingesetzt werden. Jedoch können sie potenziell zytotoxisch für lebende Mikroorganismen sein und werden bisher nur selten für Formulierungen lebender Mikroorganismen eingesetzt. Besonders vorteilhaft ist, wenn das Adjuvant nicht erst bei der Herstellung der Spritzbrühe mit dem biologischen Wirkstoff gemischt wird, sondern sich als Träger für den biologischen Wirkstoff eignet.

Das Pesticides Safety Directorate (PSD, der ausführende Bereich der Health and Safety Executive (HSE), einer

nicht-staatlichen, öffentlichen Vereinigung in Großbritannien) definiert ein Adjuvant als eine Substanz, die neben Wasser, nicht selbst als Pestizid wirksam ist, aber die Effektivität eines Pestizides erhöht (http://www.hse.gov.uk/pesticides/topics/pesticide-approvals/pesticidesregistration/applicant-guide/the-applicant-guide-adjuvan.htm). Sie bezieht sich dabei auf die VERORDNUNG (EG) Nr. 1107/2009 DES EUROPÄISCHEN PARLAMENTS UND DES RATES vom 21. Oktober 2009 über das Inverkehrbringen von Pflanzenschutzmitteln und zur Aufhebung der Richtlinien 79/117/EWG und 91/414/EWG des Rates, Artikel 2 (3)(d). Danach werden Stoffe oder Zubereitungen, die aus Beistoffen oder Zubereitungen mit einem oder mehreren Beistoffen bestehen, in der dem Verwender gelieferten Form und in Verkehr gebracht, mit der Bestimmung, vom Verwender mit einem Pflanzenschutzmittel vermischt zu werden, um dessen Wirkung oder andere pestizide Eigenschaften zu verstärken, "Zusatzstoffe" genannt. Die Begriffe "Zusatzstoffe", "Adjuvantien" oder Adjuvants werden in der vorliegenden Offenbarung synonym verwendet. Als Adjuvantien werden häufig synthetische Tenside wie z.B. ethoxylierte Alkohole, Nonylphenolethoxylate Alkylpolyglykoside oder polyethermodifizierte Trisiloxane eingesetzt.

Die biologischen Pflanzenschutzformulierungen des Standes der Technik weisen üblicherweise mehrere Nachteile auf. Generell ist für alle Formulierungen mikrobiologischer Pflanzenschutzmittel gültig, dass die enthaltenen Mikroorganismen ihre Lebensfähigkeit und/oder Keimfähigkeit mit der Zeit verlieren. Die Formulierungen müssen häufig bei Temperaturen von unter 10 °C gelagert werden, um zumindest für einige Wochen eine akzeptable Lebensfähigkeit und/oder Keimfähigkeit zu gewährleisten. Feste Formulierungen, wie WP- und WG-Formulierungen, haben zudem den Nachteil, dass für den Anwender die Gefahr des Einatmens beim Abmessen und Mischen des konzentrierten Pulvers oder Granulats besteht. Außerdem zeigen feste Formulierungen, die in Wasser dispergiert werden, häufig eine verminderte Benetzung von hydrophoben Oberflächen. Feste Formulierungen haben zudem den Nachteil, dass sie sich schlecht im Wasser verteilen und die Düsen der Sprühvorrichtung verstopfen können.

WO 2012/163322 A1 offenbart ein flüssiges Präparat für den biologischen Pflanzenschutz umfassend eine Suspension aus einem aktiven Mikroorganismums oder einer Mischung mehrerer aktiver Mikroorgansimen oder Organen von Mikroorganismen und einem polyethermodifizierten Trisiloxan als Träger. Dieses Präparat ist leicht handhabbar und zeigt eine ausreichende Lagerstabilität. Nachteilig ist hingegen, dass der Träger nicht biologisch abbaubar ist und nicht aus nachhaltigen Rohstoffen besteht und dass bei der Zerstäubung der Spritzbrühe ein großer Anteil an kleinen Tröpfchen entsteht, sodass die Spritzbrühe bei Windeinwirkung nicht zielgenau auf dem Zielsubstrat aufgebracht wird.

WO 2015/069708 A1 offenbart eine Pflanzenschutzformulierung umfassend einen Träger, einen Pilz als Pestizid und eine oberflächenaktive Substanz ausgewählt aus Sorbitanfettsäuren, Sorbitolethoxylatester, Alkoholethoxylaten und ihren Kombinationen. Als bevorzugter Träger wird Paraffinöl beschrieben. Diese Pflanzenschutzformulierungen ermöglichen eine leichtere Handhabbarkeit als feste Formulierungen, haben allerdings den Nachteil, dass sie nicht biologisch abbaubar sind.

WO 2017/210512 A1 offenbart einen nicht-wässrigen, nicht-öligen flüssigen Träger für lebende Mikroorganismen. Der Träger ist vorzugsweise ausgewählt aus der Gruppe bestehend aus Polyethylenglycol, Glyzerin, Ethylenglykol, Dipropylenglykol, Proplyencarbonat und ihren Mischungen. Dieser Träger ist im Vergleich zu festen Formulierungen leicht handhabbar und zeigt eine ausreichende Lagerstabilität. Nachteilig ist hingegen, dass die Träger keine gute Retention/Adhäsion auf der Pflanze zeigen und bei der Zerstäubung der Spritzbrühe ein großer Anteil an kleinen Tröpfchen entsteht, sodass die Spritzbrühe bei Windeinwirkung nicht zielgenau auf dem Zielsubstrat aufgebracht wird.

WO 2016/055344 A1 offenbart Zusammensetzungen, enthaltend mindestens einen hydrophoben, zumindest teilweise wasserunlöslichen Polyglycerinester in Kombination mit mindestens einem Emulgator. Konkret wird eine Mischung aus 80 Gew.-% Triglyceryltrioleat mit 20 Gew.-% Polyethylenglykol-20-Sorbitan-Trioleat beschrieben. Die Zusammensetzung wird zur Steigerung der Wirksamkeit von Pestiziden und zur Vermeidung von Spray-Drift offenbart. Es wird außerdem beschrieben, dass die Adhäsion von Sprays auf Pflanzenoberflächen verbessert wird. Der Polyglycerinester kann aus natürlichen Rohstoffen hergestellt werden und ist biologisch abbaubar. Des Weiteren ist die Zusammensetzung selbstemulgierend. Die Verwendung dieser Zusammensetzung als Träger für mikrobiologische Wirkstoffen wird allerdings nicht offenbart.

Es besteht daher weiterhin der Bedarf, Trägerzusammensetzungen für mikrobiologische Wirkstoffe bereitzustellen, welche deutliche Vorteile gegenüber dem Stand der Technik aufweisen. Diese Trägerzusammensetzungen sollen dabei vorzugsweise zudem als Adjuvantien wirken.

Die Aufgabe der vorliegenden Erfindung war es daher, neue Träger für mikrobiologische Wirkstoffe bereitzustellen, welche zumindest einen Nachteil des Standes der Technik überwinden.

Es bestand insbesondere die Aufgabe, Träger für mikrobiologische Wirkstoffe bereitzustellen, die zu einer im Vergleich zum Stand der Technik verbesserten Handhabung und Lagerfähigkeit der mikrobiologischen Wirkstoffe führen. Insbesondere soll eine im Vergleich zum Stand der Technik erhöhte biologische Wirksamkeit des mikrobiologischen Wirkstoffs erreicht werden. Es bestand also insbesondere die Aufgabe, dass die biologische Wirksamkeit und/oder Bioverfügbarkeit im Vergleich zum Stand der Technik über einen längeren Zeitraum erhalten bleibt.

Überraschenderweise wurde gefunden, dass die Verwendung einer Zusammensetzung, die einen Polyglycerinester und vorzugsweise einen Emulgator ausgewählt ist aus der Gruppe bestehend aus Sorbitanfettsäureestern und ethoxylierten Sorbitanfettsäureestern aufweist, als Träger für mikrobiologische Wirkstoffe diese Aufgabe löst.

So führt die Verwendung dieser Trägerzusammensetzung zu einer erhöhten Haltbarkeit/Lagerstabilität der mikrobiologischen Wirkstoffe, im Vergleich zu wasserdispergierbaren Pulver oder Granulaten. Gegenüber flüssigen Trägern aus dem Stand der Technik zeigen Sie überraschenderweise ein sehr gutes Anhaften der Tropfen der Spritzbrühe an Blättern. Der Träger führt zu einer guten Regenfestigkeit der Pflanzenschutzformulierung auf den Pflanzen. Die mikrobiologischen Wirkstoffe sind also auch nach einem Regen noch auf den Blättern vorhanden und wirksam. Zudem hat der Träger eine Anti-Drift-Wirkung auf das Pflanzenschutz-Spray. Die Sprühtropfen des Pflanzenschutz-Sprays werden größer und sind somit weniger anfällig gegenüber einem Abdriften während des Sprühvorgangs. Zudem ist der Träger nachhaltig aus nachwachsenden Rohstoffen herstellbar und auch weitgehend biologisch abbaubar. Der Träger zeigt also ein besonders gutes Eigenschaftsprofil.

Gelöst wird die Aufgabe der vorliegenden Erfindung daher durch die Gegenstände der unabhängigen Ansprüche. Vorteilhafte Ausgestaltungen der Erfindung sind in den nachgeordneten Ansprüchen, den Beispielen und der Beschreibung angegeben.

Die Erfindung wird nachfolgend beispielhaft beschrieben.

Sind nachfolgend Bereiche, allgemeine Formeln oder Verbindungsklassen angegeben, so sollen diese nicht nur die entsprechenden Bereiche oder Gruppen von Verbindungen umfassen, die explizit erwähnt sind, sondern auch alle Teilbereiche und Teilgruppen von Verbindungen, die durch Herausnahme von einzelnen Werten (Bereichen) oder Verbindungen erhalten werden können. Jede Ausführungsform, die durch Kombination von Bereichen/Teilbereichen und/oder Gruppen/Teilgruppen erhalten werden kann, wie beispielsweise durch Kombinationen von erfindungsgemäßen, wesentlichen, optionalen, bevorzugten, vorzugsweisen bzw. vorzugsweise ausgewählten, weiter bevorzugten, noch weiter bevorzugten, besonders bevorzugten oder insbesondere bevorzugten Bereichen/Teilbereichen und/oder Gruppen/Teilgruppen, gehört vollständig zum Offenbarungsgehalt der vorliegenden Erfindung und gilt als explizit, unmittelbar und eindeutig offenbart. Die Ausdrücke "vorzugsweise" und "bevorzugt" werden synonym verwendet. Die Ausdrücke "insbesondere" und "insbesondere bevorzugt" werden ebenfalls synonym verwendet. Werden im Rahmen der vorliegenden Beschreibung Dokumente zitiert, so soll deren Inhalt vollständig zum Offenbarungsgehalt der vorliegenden Erfindung gehören. Bei Zusammensetzungen beziehen sich die %-Angaben, wenn nicht anders angegeben, auf die Gesamtzusammensetzung. Werden nachfolgend Angaben in Prozent gemacht, so handelt es sich, wenn nicht anders angegeben, um Angaben in Gewichts-%. Werden nachfolgend Mittelwerte angegeben, so handelt es sich, wenn nicht anders angegeben, um Zahlenmittel. Werden nachfolgend Messwerte oder Stoffeigenschaften angegeben, so handelt es sich, wenn nicht anders angegeben, um Messwerte oder Stoffeigenschaften gemessen bei 25 °C sowie vorzugsweise bei einem Druck von 101325 Pa (Normaldruck). Das zahlenmittlere Molekulargewicht $M_N$ wird mittels Gel-Permeations-Chromatographie (GPC) gemäß der Norm DIN 55672:2016, vorzugsweise gemäß der Norm DIN 55672-1:2016 bestimmt. Werden nachfolgend Zahlenbereiche in der Form "von X bis Y" oder "X bis y" angegeben, wobei X und Y die Grenzen des Zahlenbereichs darstellen, so ist dies gleichbedeutend mit der Angabe "von mindestens X bis einschließlich Y", sofern nicht anders angegeben. Bereichsangaben schließen die Bereichsgrenzen X und Y also mit ein, sofern nicht anders angegeben. Wo immer Moleküle bzw. Molekülfragmente ein oder mehrere Stereozentren aufweisen oder aufgrund von Symmetrien in Isomere unterschieden werden können oder aufgrund anderer Effekte, wie beispielsweise eingeschränkter Rotation, in Isomere unterschieden werden können, sind alle möglichen Isomere von der vorliegenden Erfindung miteingeschlossen. Spezielle Ausführungen sind im Folgenden definiert, so dass Merkmale wie Indizes oder Strukturbestandteile durch die Ausführung Beschränkungen erfahren können. Für alle Merkmale, die nicht von der Beschränkung betroffen sind, bleiben die übrigen Definitionen jeweils gültig. Das Wortfragment "Poly" umfasst im Zusammenhang mit dieser Erfindung nicht nur ausschließlich Verbindungen mit zumindest 2 Wiederholungseinheiten eines oder mehrerer Monomere im Molekül, sondern vorzugsweise auch solche Zusammensetzungen von Verbindungen, die eine Molekulargewichtsverteilung aufweisen und dabei ein mittleres Molekulargewicht von mindestens 200 g/mol besitzen. Bei dieser Definition ist dem Umstand Rechnung getragen, dass es auf dem betrachteten Gebiet der Technik üblich ist, solche Verbindungen bereits als Polymere zu bezeichnen, auch wenn sie nicht einer Polymerdefinition analog OECD- oder REACH-Richtlinien zu genügen scheinen. Die verschiedenen Fragmente in den nachfolgenden Formeln (I) und (II) können statistisch verteilt sein. Statistische Verteilungen können blockweise aufgebaut sein mit einer beliebigen Anzahl an Blöcken und einer beliebigen Sequenz oder sie können einer randomisierten Verteilung unterliegen; sie können auch alternierend aufgebaut sein oder auch über die Kette, sofern eine solche vorliegt, einen Gradienten bilden, insbesondere können sie auch alle Mischformen bilden, bei denen gegebenenfalls Gruppen unterschiedlicher Verteilungen aufeinander folgen können. Die Formeln (I) und (II) beschreiben Verbindungen, die aus Wiederholungseinheiten aufgebaut sein können, wie beispielsweise sich wiederholende Fragmente, Blöcke oder Monomereinheiten, und eine Molgewichtsverteilung aufweisen können. Die Häufigkeit der Wiederholungseinheiten wird durch Indizes angegeben. Die entsprechenden Indizes sind das numerische Mittel über alle Wiederholungseinheiten. Die in den Formeln verwendeten Indizes a, b und c sind als statistische Mittelwerte (Zahlenmittel) zu betrachten. Die verwendeten Indexzahlen a, b und c sowie die Wertbereiche der angegebenen Indizes werden also als Mittelwerte der möglichen statistischen Verteilung der tatsächlichen vorhandenen Strukturen und/oder deren Mischungen verstanden. Die erfindungsgemäß zu verwendenden Polyglycerinester liegen bevorzugt in Form von equilibrierten Gemischen vor. Spezielle Ausführungen können dazu

führen, dass die statistischen Verteilungen durch die Ausführung Beschränkungen erfahren. Für alle Bereiche, die nicht von der Beschränkung betroffen sind, ändert sich die statistische Verteilung nicht. Werden im Rahmen der vorliegenden Beschreibung Dokumente zitiert, so soll deren Inhalt vollständig zum Offenbarungsgehalt der vorliegenden Erfindung gehören.

Ein erster Gegenstand der vorliegenden Erfindung ist die Verwendung einer Zusammensetzung, umfassend (insbesondere im Wesentlichen bestehend aus) mindestens einem Polyglycerinester und vorzugsweise mindestens einem Emulgator, als Träger für mindestens einen mikrobiologischen Wirkstoff, wobei der mindestens eine Emulgator ausgewählt ist aus der Gruppe bestehend aus Sorbitanfettsäureestern und ethoxylierten Sorbitanfettsäureestern.

Die als Träger zu verwendende Zusammensetzung umfassend (insbesondere im Wesentlichen bestehend aus) mindestens einem Polyglycerinester und vorzugsweise mindestens einem Emulgator, wobei der mindestens eine Emulgator ausgewählt ist aus der Gruppe bestehend aus Sorbitanfettsäureestern und ethoxylierten Sorbitanfettsäureestern, wird im Rahmen dieser Offenbarung auch einfach als Träger oder Trägerzusammensetzung bezeichnet. Eine Zusammensetzung umfassend den Träger und den mindestens einen mikrobiologischen Wirkstoff wird im Rahmen dieser Offenbarung als Wirkstoffzusammensetzung bezeichnet.

Die Trägerzusammensetzung ist vorzugsweise flüssig. Sie ermöglicht das Lösen, Suspendieren oder Dispergieren des mikrobiologischen Wirkstoffs, insbesondere von Pilzen und Pilzsporen. Der mikrobiologische Wirkstoff liegt also im Träger gelöst, suspendiert oder dispergiert vor. Der Träger unterstützt zudem das das Lösen, Suspendieren oder Dispergieren des mikrobiologischen Wirkstoffs in einer wässrigen Zusammensetzung, wie beispielsweise der Spritzbrühe.

Es ist bevorzugt, dass der mindestens eine Polyglycerinester zumindest teilweise wasserunlöslich und/oder hydrophob ist.

"Teilweise wasserunlöslich" bedeutet, dass der mindestens eine Polyglycerinester bei gegebener Temperatur in einer Konzentration von mindestens 0,01 g/l bis zu 20 g/l in Wasser bereits zu einer mit dem menschlichen Auge wahrnehmbaren Trübung führt, bevorzugt in einer Konzentration von mindestens 0,5 g/l bis zu 2 g/l zwei Phasen bildet. Vorzugsweise wird die Löslichkeit bei einer Temperatur von unter 80°C, bevorzugt unter 70, 60, 50, 40, 30, 29, 28, 27, 26, 25, 24, 23, 22, 21 und unter 20°C bestimmt. Weiterhin wird die Löslichkeit bevorzugt oberhalb von 0°C, mehr bevorzugt oberhalb von 5, 10, 15, 16, 17, 18, 19, 20, 21, 22, 23, 24 und oberhalb von 25°C bestimmt. Mehr bevorzugt wird die Löslichkeit bei Umgebungstemperatur bestimmt. Insbesondere bevorzugt wird die Löslichkeit zwischen 15°C und 30°C, mehr bevorzugt von 20°C bis zu 25°C bestimmt.

Als Maß für die Hydrophobie kann beispielsweise der HLB-Wert (HLB steht für engl. *hydrophilic lipophilic balance*) herangezogen werden. Es ist bevorzugt, dass der mindestens eine Polyglycerinester einen HLB-Wert von weniger als 8, vorzugsweise von 1 bis 7, insbesondere von 2 bis 6,5 aufweist. Der HLB-Wert kann nach verschieden Methoden des Standes der Technik bestimmt werden und ist ein anerkanntes Maß für die Hydrophobie. Bevorzugt wird der HLB-Wert nach der Methode von Griffin bestimmt (W. C. Griffin: Classification of surface active agents by HLB, J. Soc. Cosmet. Chem. 1, 1949, S. 311-326). Dabei wird der HLB-Wert gemäß der Formel

$$\mathrm{HLB} = 20 \cdot \left(1 - \frac{m_l}{m}\right)$$

berechnet, wobei $m_l$ die Molmasse des lipophilen Anteils eines Moleküls und $m$ die Molmasse des gesamten Moleküls ist. Die Molmassen werden nach den Methoden des Standes der Technik bestimmt, bevorzugt werden sie massenspektrometrisch bestimmt, die Bestimmung des lipophilen Anteils erfolgt aus den massenspektroskopischen Ergebnissen unter Anwendung der dem Fachmann bekannten stöchiometrischen Regeln. Die Molmassen können auch anhand der Molekülstruktur berechnet werden.

Bevorzugt weist der Polyglycerinester einen HLB-Wert von kleiner oder gleich 8, vorzugsweise kleiner oder gleich 7, insbesondere kleiner oder gleich 6,5 auf. Weiterhin bevorzugt beträgt der HLB-Wert mindestens 0,5, vorzugsweise mindestens 1 und insbesondere mindestens 2. Es ist daher auch bevorzugt, dass der mindestens eine Polyglycerinester einen HLB-Wert von 0,5 bis 8, vorzugsweise von 1 bis 7, insbesondere von 2 bis 6,5 aufweist.

Es ist bevorzugt, dass die Acyloxyreste (auch als Alkanoyloxyreste bezeichnet) des mindestens einen Polyglycerinesters 4 bis 40, vorzugsweise 8 bis 22, insbesondere 10 bis 18 Kohlenstoffatomen aufweisen.

Es ist weiterhin bevorzugt, dass der mindestens eine Polyglycerinester eine Verbindung der allgemeinen Formel (I) ist,

$M_a\ D_b\ T_c$ Formel (I);

mit:

$M = [C_3H_5(OR)_2O_{1/2}]$;

$D = [C_3H_5(OR)_1O_{2/2}]$;
$T = [C_3H_5O_{3/2}]$;
a = 1 bis 10, vorzugsweise 2 bis 3, insbesondere 2;
b = 0 bis 10, vorzugsweise größer 0 bis 5, insbesondere 1 bis 3;
c = 0 bis 3, vorzugsweise 0 bis 1, insbesondere 0;
mit der Maßgabe, dass gilt:

a + b + c = 2 bis 20, vorzugsweise 2 bis 4, insbesondere 3;
wobei die Reste R jeweils unabhängig voneinander ausgewählt sind aus der Gruppe bestehend aus Acylresten R'-C(=O)- und H, mit der Maßgabe, dass mindestens ein Rest R ungleich H ist; wobei die Reste R' jeweils unabhängig voneinander ausgewählt sind aus der Gruppe bestehend aus einbindigen aliphatischen, gesättigten oder ungesättigten Kohlenwasserstoffresten mit 3 bis 39, vorzugsweise 7 bis 21, insbesondere mit 9 bis 17 Kohlenstoffatomen.

Vorzugsweise gilt für die Einheiten M, D und T:

M = 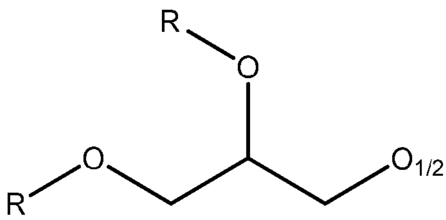 ;

D = 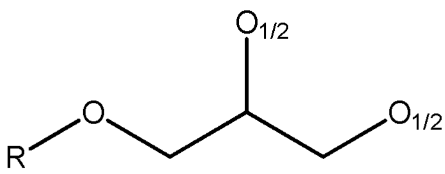 und/oder 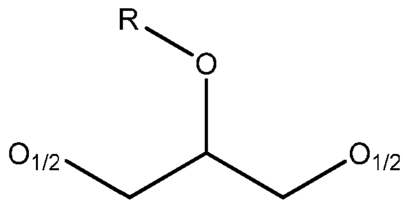 ;

T = 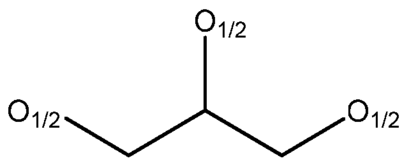 .

Es ist weiter bevorzugt, dass der mindestens eine Polyglycerinester eine Verbindung der allgemeinen Formel (II) ist,

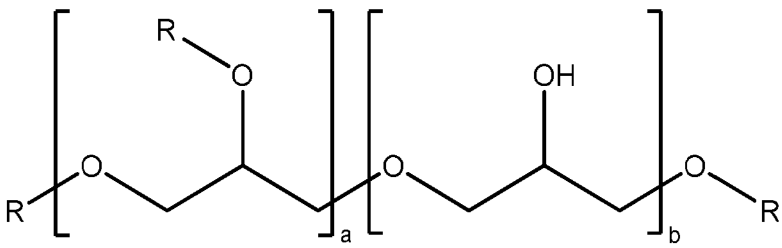 Formel (II);

wobei

a = 1 bis 10, vorzugsweise 2 bis 3, insbesondere 2;
b = 0 bis 10, vorzugsweise größer 0 bis 5, insbesondere 1 bis 3;
mit der Maßgabe, dass gilt:

a + b = 2 bis 20, vorzugsweise 2 bis 4, insbesondere 3;

wobei die Reste R jeweils unabhängig voneinander ausgewählt sind aus der Gruppe bestehend aus Acylresten R'-C(=O)- und H, mit der Maßgabe, dass mindestens ein Rest R ungleich H ist; wobei die Reste R' jeweils unabhängig voneinander ausgewählt sind aus der Gruppe bestehend aus einbindigen aliphatischen, gesättigten oder ungesättigten Kohlenwasserstoffresten mit 3 bis 39, vorzugsweise 7 bis 21, insbesondere mit 9 bis 17 Kohlenstoffatomen.

Bevorzugt weisen die Polyglycerinester der erfindungsgemäßen Zusammensetzungen mehr als einen Rest R der Form R'-C(=O)-, mehr bevorzugt mindestens 2, weiter mehr bevorzugt mindestens 3 auf.

Die Reste R der Form R'-C(=O)- sind bevorzugt die Acylreste von gesättigten oder ungesättigten Fettsäuren, wobei die Fettsäuren 4 bis zu 40 Kohlenstoffatome aufweisen, mehr bevorzugt Buttersäure (Butansäure), Capronsäure (Hexansäure), Caprylsäure (Octansäure), Caprinsäure (Decansäure), Laurinsäure (Dodecansäure), Myristinsäure (Tetradecansäure), Palmitinsäure (Hexadecansäure), Stearinsäure (Octadecansäure), Arachinsäure (Eicosansäure), Behensäure (Docosansäure), Lignocerinsäure (Tetracosansäure), Palmitölsäure ((Z)-9-Hexadecensäure), Ölsäure ((Z)-9-Hexadecensäure), Elaidinsäure ((E)-9-Octadecensäure), cis-Vaccensäure ((Z)-11-Octadecensäure), Linolsäure ((9Z,12Z)-9,12-Octadecadiensäure), alpha-Linolensäure ((9Z,12Z,15Z)-9,12,15-Octadecatriensäure), gamma-Linolensäure ((6Z,9Z,12Z)-6,9,12-Octadecatriensäure), Di-homo-gamma-Linolensäure ((8Z,11Z,14Z)-8,11,14-Eicosatriensäure), Arachidonsäure ((5Z,8Z,11Z,14Z)-5,8,11,14- Eicosatetraensäure), Erucasäure ((Z)-13-Docosensäure), Nervonsäure ((Z)-15-Tetracosensäure), Rizinolsäure, Hydroxystearinsäure und Undecylensäure, sowie deren Mischungen, wie beispielsweise Rapsölsäure, Sojafettsäure, Sonnenblumenfettsäure, Erdnussfettsäure und Tallölfettsäure. Besonders bevorzugt sind hierbei Reste von Ölsäure.

Bei der Berechnung des HLB-Werts ergibt sich die Molmasse des lipophilen Molekülteils aus dem arithmetischen Mittel der Summe der Molmassen aller im Molekül als Teile der Acylreste R'-C(=O)-vorhandenen Reste R'.

Quellen für geeignete Fettsäuren oder Fettsäureester, besonders Glyceride, können pflanzliche oder tierische Fette, Öle oder Wachse sein. Beispielsweise können verwendet werden: Schweineschmalz, Rindertalg, Gänsefett, Entenfett, Hühnerfett, Pferdefett, Walöl, Fischöl, Palmöl, Olivenöl, Avokadoöl, Samenkernöle, Kokosöl, Palmkernöl, Kakaobutter, Baumwollsamenöl, Kürbiskernöl, Maiskeimöl, Sonnenblumenöl, Weizenkeimöl, Traubenkernöl, Sesamöl, Leinsamenöl, Sojabohnenöl, Erdnussöl, Lupinöl, Rapsöl, Senföl, Rizinusöl, Jatropaöl, Walnussöl, Jojobaöl, Lecithin z.B. auf Basis von Soja, Raps, oder Sonnenblumen, Knochenöl, Klauenöl, Borretschöl, Lanolin, Emuöl, Hirschtalg, Murmeltieröl, Nerzöl, Distelöl, Hanföl, Kürbisöl, Nachtkerzenöl, Tallöl, sowie Carnaubawachs, Bienenwachs, Candelillawachs, Ouricuriwachs, Zuckerrohrwachs, Retamowachs, Carandaywachs, Raffiawachs, Espartowachs, Alfalfawachs, Bambuswachs, Hempwachs, Douglastannenwachs, Korkwachs, Sisalwachs, Flachswachs, Baumwollwachs, Dammarwachs, Teewachs, Kaffeewachs, Reiswachs, Oleanderwachs oder Wollwachs.

Besonders bevorzugt sind die Polyglycerinester Verbindungen der Formeln (I) oder (II) mit im arithmetischen Mittel 2,9 bis 3,1 Resten der Form R'-C(=O)- und einem HLB-Wert von 4 bis 6,5.

Weiterhin besonders bevorzugt sind die Polyglycerinester Verbindungen der Formel (II), wobei die Summe a + b gleich 3 ist und die im arithmetischen Mittel 2,9 bis 3,1 Reste der Form R'-C(=O)- und einen HLB-Wert von 4 bis 6,5 aufweisen.

Weiterhin besonders bevorzugt sind die Polyglycerinester Verbindungen der Formel (II), die im arithmetischen Mittel 2,9 bis 3,1 Reste der Form R'-C(=O)- und einen HLB-Wert von 4 bis 6,5 aufweisen, wobei die Acylreste von Fettsäuregemischen enthaltend Ölsäure, Stearinsäure, Palmitinsäure und gamma-Linolensäure sind und wobei bevorzugt besagte Fettsäuren mindestens 85 Gew.-% im Fettsäuregemisch ausmachen.

Besonders bevorzugt sind die Polyglycerinester Verbindungen der Formel (II), die im arithmetischen Mittel 2,9 bis 3,1 Reste der Form R'-C(=O)- und einen HLB-Wert von 4 bis 6,5 aufweisen, wobei die Acylreste von Fettsäuregemischen enthaltend Ölsäure, Stearinsäure, Palmitinsäure und gamma-Linolensäure stammen und wobei bevorzugt besagte Fettsäuren mindestens 85 Gew.-% im Fettsäuregemisch ausmachen.

Insbesondere bevorzugt sind die Polyglycerinester Verbindungen der Formel (II), die im arithmetischen Mittel 2,9 bis 3,1 Reste der Form R'-C(=O)- und einen HLB-Wert von 4 bis 6,5 aufweisen, wobei der Massenanteil von Ölsäure-Acylresten mindestens 75 %, vorzugsweise 85 %, insbesondere 95 % bezogen auf die Masse aller Acylreste beträgt.

Es ist besonders bevorzugt, dass der mindestens eine Polyglycerinester Triglycerintrioleat ist.

Bevorzugt weist der mindestens eine Polyglycerinester bzw. der Träger, umfassend den mindestens einen Polyglycerinester und vorzugsweise den mindestens einen Emulgator, eine biologische Abbaubarkeit von mindestens 50%, vorzugsweise mindesten 55%, insbesondere mindestens 60% auf, wobei der Höchstwert der biologischen Abbaubarkeit bei 100% liegt.

Es ist bevorzugt, dass der Träger zusätzlich mindestens einen Emulgator enthält. Es ist aber nicht notwendig, dass der Träger mindestens einen Emulgator enthält.

Der Emulgator ist von dem mindestens einen Polyglycerinester unterschiedlich.

Erfindungsgemäß ist der mindestens eine Emulgator ausgewählt ist aus der Gruppe bestehend aus Sorbitanfettsäureestern und ethoxylierten Sorbitanfettsäureestern, vorzugsweise ethoxylierten Sorbitanfettsäureestern.

Es ist weiter bevorzugt, dass die Acyloxyreste des mindestens einen Sorbitanfettsäureesters beziehungsweise ethoxylierten Sorbitanfettsäureesters 4 bis 40, vorzugsweise 8 bis 22, insbesondere 10 bis 18 Kohlenstoffatome aufweisen und/oder dass der mindestens eine Sorbitanfettsäureester beziehungsweise ethoxylierte Sorbitanfettsäureester 0 bis 40, vorzugsweise 10 bis 30, insbesondere 15 bis 25 Oxyethlyen-Gruppen aufweist.

Die Fettsäuren bzw. Fettsäurereste der Sorbitanfettsäureester sind bevorzugt wie die Fettsäuren bzw. Fettsäurereste der Polyglycerinester definiert. Vorzugsweise stammen die Acylreste (auch als Alkanoylreste bezeichnet) von Fettsäuregemischen enthaltend Ölsäure, Stearinsäure, Palmitinsäure und gamma-Linolensäure, wobei bevorzugt besagte Fettsäuren mindestens 85 Gew.-% im Fettsäuregemisch ausmachen. Insbesondere bevorzugt sind ethoxylierte Sorbitanfettsäureester, wobei der Massenanteil von Ölsäure-Acylresten mindestens 75 %, vorzugsweise 85 %, insbesondere 95 % bezogen auf die Masse aller Acylreste beträgt.

Es ist bevorzugt, dass der mindestens eine Emulgator einen HLB-Wert von größer oder gleich 9, vorzugsweise von größer oder gleich 10, insbesondere von größer oder gleich 11 aufweist. Weiterhin bevorzugt beträgt der HLB-Wert maximal 16, vorzugsweise maximal 15, insbesondere maximal 13. Es ist daher auch bevorzugt, dass der mindestens eine Emulgator einen HLB-Wert von 9 bis 16, vorzugsweise von 10 bis 15, insbesondere von 11 bis 13 aufweist. Der HLB-Wert wird wie oben beschrieben bestimmt. Der HLB-Wert der Sorbitanfettsäureester und/oder ethoxylierten Sorbitanfettsäureester wird vorzugsweise wie für die Polyglycerinester bestimmt. Die Molmasse des lipophilen Molekülteils ergibt sich aus dem arithmetischen Mittel der Summe der Molmassen aller im Molekül als Bestandteil der Acylreste R'-(CO)- vorhandenen Reste R'. Die Reste R' sind dabei vorzugsweise wie für die Polyglycerinester definiert. Der Rest R' als Bestandteil eines Acylrestes R'-(CO)- des Sorbitanfettsäureesters bzw. ethoxylierten Sorbitanfettsäureesters ist dabei bevorzugt ausgewählt aus der Gruppe bestehend aus einbindigen aliphatischen, gesättigten oder ungesättigten Kohlenwasserstoffresten mit 3 bis 39, vorzugsweise 7 bis 21, insbesondere mit 9 bis 17 Kohlenstoffatomen. Die Berechnung der Molmasse des gesamten Moleküls wird wie oben definiert durchgeführt.

Es ist besonders bevorzugt, dass der mindestens eine Emulgator Polyethylenglykol-20-SorbitanTrioleat ist. Die Zahl 20 gibt die mittlere Anzahl an Ethylenoxid-Einheiten im Polyethlyenglykol-Rest an.

Der HLB-Wert des mindestens einen Polyglycerinesters und des mindestens einen Emulgators sind vorzugsweise aufeinander abgestimmt. Es ist dabei bevorzugt, dass der Polyglycerinester einen HLB-Wert von kleiner oder gleich 8, vorzugsweise kleiner oder gleich 7, insbesondere kleiner oder gleich 6,5 und der mindestens eine Emulgator einen HLB-Wert von größer oder gleich 9, vorzugsweise von größer oder gleich 10, insbesondere von größer oder gleich 11 aufweist. Es ist weiter bevorzugt, dass der mindestens eine Polyglycerinester einen HLB-Wert von 0,5 bis 8, vorzugsweise von 1 bis 7, insbesondere von 2 bis 6,5 aufweist und der mindestens eine Emulgator einen HLB-Wert von 9 bis 16, vorzugsweise von 10 bis 15, insbesondere von 11 bis 13 aufweist.

Insbesondere bevorzugt ist, dass der mindestens eine Polyglycerinester Triglycerintrioleat und der mindestens eine Emulgator Polyethylenglykol-20-Sorbitan-Trioleat ist. Die Kombination aus Triglycerintrioleat und Polyethylenglykol-20-Sorbitan-Trioleat zeigt besonders vorteilhafte Eigenschaften als Träger für einen mikrobiologischen Wirkstoff.

Der Träger besteht vorzugsweise zu einem überwiegenden Teil aus dem mindestens einen Polyglycerinester und, falls zusätzlich enthalten, dem mindestens einem Emulgator. Es ist bevorzugt, dass der Massenanteil des mindestens einen Polyglycerinesters zusammen mit dem gegebenenfalls zusätzlich enthaltenen mindestens einen Emulgator mindestens 90 %, bevorzugt mindestens 95 %, insbesondere mindestens 99 % bezogen auf die Gesamtmasse des Trägers beträgt. Es ist besonders vorteilhaft, wenn die als Träger verwendete Zusammensetzung aus dem mindestens einen Polyglycerinester und gegebenenfalls zusätzlich dem mindestens einen Emulgator (im Wesentlichen) besteht.

Vorzugsweise gilt, dass der Massenanteil des mindestens einen Polyglycerinesters bezogen auf die Gesamtmasse der Trägerzusammensetzung 60 % bis 100 %, vorzugsweise 70 % bis 90 %, insbesondere 75 % bis 85 % beträgt; und der Massenanteil des gegebenenfalls zusätzlich enthaltenen mindestens einen Emulgators bezogen auf die Gesamtmasse der Trägerzusammensetzung 0 % bis 40 %, vorzugsweise 10 % bis 30 %, insbesondere 15 % bis 25 % beträgt. Vorzugsweise besteht die Trägerzusammensetzung im Wesentlichen aus dem mindestens einen Polyglycerinester und gegebenenfalls zusätzlich dem mindestens einen Emulgator.

Weiter bevorzugt gilt, dass der Träger den mindestens einen Polyglycerinester in einem Massenanteil bezogen auf die Gesamtmasse der Trägerzusammensetzung von 60 % bis 100 %, vorzugsweise von 70 % bis 90 %, insbesondere von 75 % bis 85 % enthält, und dass der Träger als den mindestens einen Emulgator mindestens einen Sorbitanfettsäureester und/oder mindestens einen ethoxylierten Sorbitanfettsäureester, vorzugsweise mindestens einen ethoxylierten Sorbitanfettsäureester, in einem Massenanteil bezogen auf die Gesamtmasse der Trägerzusammensetzung von 0 % bis 40 %, vorzugsweise von 10 % bis 30 %, insbesondere von 15 % bis 25 % enthält.

Weiter bevorzugt gilt, dass der Träger als Polyglycerinester Triglycerintrioleat in einem Massenanteil bezogen auf die Gesamtmasse der Trägerzusammensetzung von 60 % bis 100 %, vorzugsweise von 70 % bis 90 %, insbesondere von 75 % bis 85 % enthält, und dass der Träger als Emulgator Polyethylenglykol-20-Sorbitan-Trioleat in einem Massenanteil bezogen auf die Gesamtmasse der Trägerzusammensetzung von 0 % bis 40 %, vorzugsweise von 10 % bis 30 %, insbesondere von 15 % bis 25 % enthält.

Weiter bevorzugt gilt, dass der Träger im Wesentlichen aus dem mindestens einem Polyglycerinester in einem

Massenanteil bezogen auf die Gesamtmasse der Trägerzusammensetzung von 60 % bis 100 %, vorzugsweise von 70 % bis 90 %, insbesondere von 75 % bis 85 % und dem mindestens einen Emulgator ausgewählt aus der Gruppe bestehend aus Sorbitanfettsäureestern und ethoxylierten Sorbitanfettsäureestern, vorzugsweise ethoxylierten Sorbitanfettsäureestern, in einem Massenanteil bezogen auf die Gesamtmasse der Trägerzusammensetzung von 0 % bis 40 %, vorzugsweise von 10 % bis 30 %, insbesondere von 15 % bis 25 % besteht.

Noch weiter bevorzugt gilt, dass der Träger im Wesentlichen aus Triglycerintrioleat in einem Massenanteil bezogen auf die Gesamtmasse der Trägerzusammensetzung von 60 % bis 100 %, vorzugsweise von 70 % bis 90 %, insbesondere von 75 % bis 85 % und Polyethylenglykol-20-Sorbitan-Trioleat in einem Massenanteil bezogen auf die Gesamtmasse der Trägerzusammensetzung von 0 % bis 40 %, vorzugsweise von 10 % bis 30 %, insbesondere von 15 % bis 25 % besteht.

Im Rahmen der Erfindung ist der mikrobiologische Wirkstoff ausgewählt aus der Gruppe bestehend aus Mikroorganismen, Organen von Mikroorganismen und ihren Mischungen. Es ist insbesondere bevorzugt, dass der Mikroorganismus lebt und/oder aktiv ist.

Es weiterhin bevorzugt, dass der mikrobiologische Wirkstoff eine gegen einen bestimmten Krankheitserreger, vorzugsweise Pflanzenkrankheitserreger, gerichtete, vorzugsweise antagonistische und/oder hyperparasitäre Wirkung aufweist.

Es ist bevorzugt, dass der mikrobiologische Wirkstoff ausgewählt ist aus der Gruppe bestehend aus Akariziden (AC), Bakteriziden (BA), Fungiziden (FU), Herbiziden (HE), Insektiziden (IN), Nematiziden (NE), Wachstumsregulatoren (PG), Pflanzenstärkungsmittel (PS), Biostimulanzien, Inokulaten oder ihren Mischungen. Einige dieser biologischen Wirkstoffe sind beispielsweise in The Manual of Biocontrol Agents, 2001, The British Crop Protection Council angegeben. Die vorliegende Erfindung beschränkt sich jedoch nicht nur auf diese dort aufgeführten Wirkstoffe. Vorzugsweise erhöht der mikrobiologische Wirkstoff die Resistenz und/oder Stresstoleranz und/oder Nährstoffverfügbarkeit bei Pflanzen.

Zu den Mikroorganismen zählen im Sinne der vorliegenden Offenbarung Bakterien, Pilze, Algen, Protozoen und Viren.

Die Mikroorganismen werden demnach ausgewählt aus der Gruppe bestehend aus Bakterien, Pilzen, Algen, Protozoen, Viren und ihren Mischungen.

Vorzugsweise wird der Mikroorganismus ausgewählt aus der Gruppe bestehend aus Pilzen und Bakterien.

In einer bevorzugten Ausführungsform wird der Mikroorganismus nicht aus der Gruppe der Viren ausgewählt, insbesondere nicht aus der aus der Gruppe bestehend aus Viren, Algen und Protozoen.

In einer bevorzugten Ausführungsform wird der mikrobiologische Wirkstoff ausgewählt aus der Gruppe bestehend aus Pilzen, Pilzorganen, Bakterien, Bakterienorganen und ihren Mischungen.

In einer bevorzugten Ausführungsform wird der mikrobiologische Wirkstoff ausgewählt aus der Gruppe bestehend aus Pilzen, Pilzorganen und ihren Mischungen.

Es ist weiterhin bevorzugt, dass die Pilzorgane ausgewählt werden aus der Gruppe bestehend aus Sporen, Konidien, Blastosporen, Chlamydosporen, Sklerotien, Hyphensegmente und ihren Mischungen.

Weiter bevorzugt wird der mikrobiologische Wirkstoff ausgewählt aus der Gruppe bestehend aus den Pilzen *Ampelomyces quisqualis, Aureobasidium pullulans, Beauveria bassiana, Beauveria brongniartii, Candida oleophila, Clonostachys rosea*, *Coniothyrium minitans, Gliocladium catenulatum, Gliocladium virens, Isaria fumosorosea*, *Isaria* spp., *Laetisaria arvalis, Lecanicillium lecanii, Lecanicillium muscarium, Metarhizium anisopliae, Myrothecium verrrucaria, Metarhizium riley (Nomuraea rileyi), Paecilomyces lilacinus, Phlebiopsis gigantea, Phoma macrostoma, Purpureocillium lilacinus, Pythium oligandrum, Talaromyces flavus, Teratospema oligociadum, Trichoderma asperellum, Trichoderma atroviride, Trichoderma gamsii, Trichoderma hamatum, Trichoderma harzianum, Trichoderma koningii, Trichoderma reesei, Trichoderma* spp., *Verticillium biguttatum,* ihren Pilzorganen und Mischungen dieser Pilze und/oder Pilzorgane.

Besonders bevorzugt wird der mikrobiologische Wirkstoff ausgewählt aus der Gruppe bestehend aus den Pilzen *Ampelomyces quisqualis, Aureobasidium pullulans, Beauveria bassiana, Candida oleophila, Clonostachys rosea, Coniothyrium minitans, Gliocladium virens, Isaria fumosorosea, Lecanicillium muscarium, Metarhizium anisopliae, Myrothecium verrrucaria, Metarhizium rileyi (Nomuraea rileyi), Purpureocillium lilacinus, Phlebiopsis gigantea, Trichoderma asperellum, Trichoderma atroviride, Trichoderma gamsii, Trichoderma hamatum, Trichoderma harzianum, Trichoderma koningii, Trichoderma reesei,* ihren Pilzorganen und Mischungen dieser Pilze und/oder Pilzorgane.

Der Einsatz folgender Pilze mit gegen bestimmte Pflanzenkrankheitserreger antagonistischer und/oder hyperparasitärer Wirkung ist besonders bevorzugt: *Ampelomyces quisqualis, Beauveria bassiana, Beauveria brongniartii, Clonostachys rosea, Coniothyrium minitans, Gliocladium catenulatum, Isaria* spp., *Laetisaria arvalis, Lecanicillium lecanii, Lecanicillium muscarium, Metarhizium anisopliae, Metarhizium rileyi (Nomuraea rileyi), Paecilomyces lilacinus, Phoma macrostoma, Pythium oligandrum, Talaromyces flavus, Teratosperma oligociadum, Trichoderma* spp. und *Verticillium biguttatum.*

Als Pilze, welche die Nährstoffverfügbarkeit im Boden verbessern bzw. die Widerstandsfähigkeit der Pflanzen gegenüber Stressfaktoren (auch Krankheits- und Schaderreger) erhöhen, werden bevorzugt eingesetzt: *Penicillium bilaii, Trichoderma* spp. sowie alle Arten, die in die Gruppe der *Mykorrhizapilze* eingeordnet werden können.

Mikrobiologische Wirkstoffe, ausgewählt der Gruppe bestehend aus Pilzen, Pilzorganen und ihren Mischungen, sind besonders für die Verwendung als Pflanzenschutzmittel, für die Verwendung als Biostimulanz und/oder für die Behandlung von Saatgut geeignet.

In einer weiteren bevorzugten Ausführungsform ist der mikrobiologische Wirkstoff ein Bakterium oder eine Mischung aus verschiedenen Bakterien.

In einer weiteren bevorzugten Ausführungsform ist das Bakterium oder die Mischung aus verschiedenen Bakterien ausgewählt aus der Gruppe bestehend aus *Azospirillum brasilense, Azotobacter chroococcum, Bacillus amyloliquefaciens, Bacillus firmus, Bacillus licheniformis, Bacillus mycoides, Bacillus pumilus, Bacillus subtilis, Bacillus thuringiensis, Bradyrhizobium* spp., *Burkholderia* spp., *Chromobacterium subtsugae, Gluconacetobacter* spp., *Pseudomonas chlororaphis, Pseudomonas fluorescens, Pseudomonas syringae, Rhizobium* spp., *Streptomyces griseoviridis, Streptomyces lydicus* und ihren Mischungen. Diese Zusammensetzungen sind besonders für die Verwendung als Pflanzenschutzmittel, für die Verwendung als Biostimulanz und/oder für die Behandlung von Saatgut geeignet.

In einer weiteren bevorzugten Ausführungsform ist das Bakterium oder die Mischung aus verschiedenen Bakterien ausgewählt aus der Gruppe bestehend aus *Lactobacillus gasseri, Lactobacillus paracasei, Lactobacillus plantarum, Lactobacillus crispatus, Lactobacillus casei, Lactobacillus animalis, Lactobacillus rhamnosus, Lactobacillus pentosus, Lactobacillus reuteri, Lactococcus lactis, Bacillus pumilus, Bacillus licheniformis, Bacillus coagulans, Bacillus cereus, Bacillus subtilis, Bacillus amyloliquefaciens, Clostridium butyricum, Enterococcus faecium, Streptococcus faecium, Lactobacillus acidophilus, Lactobacillus salivarius, Lactobacillus fermentum, Lactobacillus johnsonii. Lactobacillus helveticus, Streptococcus thermophiles, Pediococcus acidilactici, Bifidobacterium lactis, Bifidobacterium adolescentis, Bifidobacterium lactobacillus, Bifidobacterium animalis, Bifidobacterium longum, Bifidobacterium infantis* und ihren Mischungen. Diese Zusammensetzungen eignen sich besonders für die Verwendung als Probiotikum in Nahrungs- und/oder Futtermitteln.

In einer weiteren bevorzugten Ausführungsform ist der mikrobiologische Wirkstoff ausgewählt aus der Gruppe bestehend aus Laktobazillen, Bifidobakterien, *Enterococcus faecalis, Enterococcus faecium* sowie den Hefepilzen *Saccharomyces boulardii* und *Saccharomyces cerevisiae* und ihren Mischungen. Diese Zusammensetzungen können beispielweise zur Verwendung als probiotisches Arzneimittel geeignet sein. Für einige Krankheiten und Anwendungsgebiete ist die Wirksamkeit von probiotischen Arzneimittel verhältnismäßig gut erforscht. Dazu zählen verschiedene chronischentzündliche Darmerkrankungen, verschiedene Durchfallerkrankungen, chronische Verstopfung, Vorbeugung vor Allergien und Infektionen Frühgeborener, Vorbeugung vor Neurodermitis, Infekte von Hals, Nase, Ohren, Harnwegsinfekte und Zahnkaries.

In einer weiteren bevorzugten Ausführungsform der Zusammensetzung ist der mikrobiologische Wirkstoff ein Virus oder eine Mischung verschiedener Viren, vorzugsweise ausgewählt aus der Gruppe der Baculoviren, weiter bevorzugt der Gattungen *Nucleopolyhedrovirus* und *Granulovirus.*

In einer bevorzugten Ausführungsform der Zusammensetzung ist als mikrobiologischer Wirkstoff das Virus CpGV (*Cydia pomonella granulovirus*) ausgewählt. Dieses Virus wird beispielsweise zum Schutz vor Raupen des Apfelwicklers im Obstbau eingesetzt. In einer weiteren bevorzugten Ausführungsform der Zusammensetzung ist als mikrobiologischer Wirkstoff der Virus HearNPV (*Helicoverpa armigera Nucleopolyhedrovirus*) ausgewählt. Dieses Virus wirkt spezifisch gegen die Larven des Baumwollkapselwurms und wird beispielsweise zum Schutz von Baumwollpflanzen eingesetzt.

Weiterhin gemäß der Erfindung bevorzugt ist, dass der mikrobiologische Wirkstoff eine Mischung der oben genannten Mikroorganismen und/oder ihren Organen ist.

Es ist weiterhin bevorzugt, dass der mindestens eine mikrobiologische Wirkstoff ausgewählt ist aus der Gruppe bestehend aus *Trichoderma harzianum, Bacillus amyloliquefaciens, Beauveria bassiana, Metarhizium rileyi (Nomuraea rileyi), Metarhizium anisopliae, Clonostachys rosea, Aureobasidium pullulans, Coniothyrium minitans* und ihren Organen, wobei die Organe vorzugsweise ausgewählt sind aus der Gruppe bestehend aus Sporen, Konidien, Blastosporen, Chlamydosporen, Sklerotien und Hyphensegmenten.

Es ist weiterhin bevorzugt, dass der mindestens eine mikrobiologische Wirkstoff Sporen, vorzugsweise Pilzsporen und/oder Bakteriensporen umfasst, insbesondere Sporen von *Trichoderma harzianum* und/oder von *Bacillus amyloliquefaciens* und/oder von *Beauveria bassiana* und/oder von *Metarhizium rileyi (Nomuraea rileyi)* und/oder von *Metarhizium anisopliae* und/oder von *Clonostachys rosea* und/oder von *Aureobasidium pullulans* und/oder von *Coniothyrium minitans.*

Es ist noch mehr bevorzugt, dass der mindestens eine mikrobiologische Wirkstoff ausgewählt ist aus der Gruppe bestehend aus *Trichoderma harzianum* und Sporen von *Trichoderma harzianum.*

Es ist besonders bevorzugt, dass es sich bei dem mindestens einen mikrobiologischen Wirkstoff um Sporen von Trichoderma harzianum handelt. Es ist also besonders bevorzugt, dass der mindestens eine mikrobiologische Wirkstoff Sporen von Trichoderma harzianum umfasst oder aus ihnen besteht.

Bevorzugt ist auch, dass der mikrobiologische Wirkstoff vegetative Zellen umfasst, insbesondere vegetative Zellen von *Pseudomonas fluorescens* und/oder *Pseudomonas chlororaphis.*

Durch die Einstellung der Wasseraktivität wird die Lebensfähigkeit und/oder Keimfähigkeit des enthaltenen mikrobiellen Wirkstoffs erhöht und somit auch dessen Lagerfähigkeit (Haltbarkeit). Die Wasseraktivität *(activity of water, $a_w$)* ist ein thermodynamischer Parameter. Sie ist ein Maß für die für chemische, biochemische und mikrobielle Reaktionen zur Verfügung stehende Wassermenge von Proben, wie beispielsweise wässrigen Lösungen und Lebensmitteln, und lässt sich auch zur Charakterisierung der Trägerzusammensetzungen einsetzen. Die Wasseraktivität wird als $a_w$-Wert angegeben und ist definiert als Verhältnis des Wasserdampfdrucks über der Probe (p) zum Wasserdampfdruck reinen Wassers ($p_0$) bei der gleichen Temperatur, $a_w = p/p_0$. Die Wasseraktivität entspricht 1/100 der relativen Gleichgewichtsfeuchtigkeit (RGF). Die relative Gleichgewichtsfeuchtigkeit wird auch als Gleichgewichtsfeuchte (*equilibrium relative humidity,* ERH) bezeichnet. Reines Wasser hat einen $a_w$-Wert von 1 und jeder Zusatz von wasserbindenden Substanzen bewirkt eine Absenkung des $a_w$-Wertes unter 1. Es ist bevorzugt, dass der $a_w$-Wert der Trägerzusammensetzung weniger als 0,4, vorzugsweise weniger als 0,3, insbesondere weniger als 0,25 beträgt. Methoden zur Bestimmung des $a_w$-Wertes sind dem Fachmann bekannt. Der $a_w$-Wert wird vorzugsweise wie in den Beispielen beschrieben bestimmt.

Synthesebedingt können Polyglycerinester Restmengen von Wasser enthalten. Es kann daher von Vorteil sein, den Wassergehalt und somit die Wasseraktivität einzustellen, insbesondere zu verringern. Dies kann beispielsweise mittels eines thermischen Trennverfahrens geschehen. Thermische Trennverfahren sind unter diesem Begriff dem Fachmann bekannt und umfassen alle Verfahren, die auf der Einstellung eines thermodynamischen Phasengleichgewichtes beruhen. Bevorzugte thermische Trennverfahren sind ausgewählt aus der Gruppe bestehend aus Destillation, Rektifikation, Adsorption, Kristallisation, Extraktion, Absorption, Trocknung und Ausfrieren, besonders bevorzugt sind Methoden der Destillation und Rektifikation. Zur Trocknung können dabei auch Trocknungsmittel wie Molekularsiebe, z.B. Zeolithe, eingesetzt werden.

Die erfindungsgemäße Verwendung der Trägerzusammensetzung führt dabei zu einer Steigerung der Lagerstabilität des mikrobiologischen Wirkstoffs. Vorzugsweise wird die Lagerstabilität wie in den Beispielen beschrieben bestimmt.

Es ist daher weiterhin bevorzugt, dass der Anteil keimfähiger Sporen bzw. vegetativer Zellen nach Lagerung, bestimmt wie in den Beispielen beschrieben, nach 28 Tagen mindestens 6 %, weiter bevorzugt mindestens 8 %, insbesondere mindestens 10 % bezogen auf den Startwert beträgt.

Ein weiterer Gegenstand der Erfindung ist daher die erfindungsgemäße Verwendung der Trägerzusammensetzung zur Steigerung der Lagerstabilität des mikrobiologischen Wirkstoffs.

Noch ein weiterer Gegenstand der Erfindung ist daher ein Verfahren zur Steigerung der Lagerstabilität mindestens eines mikrobiologischen Wirkstoffs durch Verwendung einer Zusammensetzung, umfassend mindestens einen Polyglycerinester und vorzugsweise mindestens einen Emulgator wobei der mindestens eine Emulgator ausgewählt ist aus der Gruppe bestehend aus Sorbitanfettsäureestern und ethoxylierten Sorbitanfettsäureestern.

Für den Träger, den mindestens einen Polyglycerinester, den mindestens einen Emulgator und den mikrobiologischen Wirkstoff gelten dabei die obigen Ausführungen. Alle Definitionen, Ausführungsformen und Erläuterungen, die für die erfindungsgemäße Verwendung gelten, gelten also *mutatis mutandis* auch für das erfindungsgemäße Verfahren und umgekehrt.

Ein weiterer Gegenstand der Erfindung ist eine Zusammensetzung (auch als Wirkstoffzusammensetzung bezeichnet), umfassend

(a) einen Träger, umfassend

(a1) mindestens einen Polyglycerinester und vorzugsweise
(a2) mindestens einen Emulgator, ausgewählt aus der Gruppe bestehend aus Sorbitanfettsäureestern und ethoxylierten Sorbitanfettsäureestern,

und

(b) mindestens einen mikrobiologischen Wirkstoff,

wobei der $a_w$-Wert der Wirkstoffzusammensetzung weniger als 0,4, vorzugsweise weniger als 0,3, insbesondere weniger als 0,25 beträgt.

Für den Träger, den mindestens einen Polyglycerinester, den mindestens einen Emulgator und den mikrobiologischen Wirkstoff gelten die obigen Ausführungen. Alle Definitionen, Ausführungsformen und Erläuterungen, die für die erfindungsgemäße Verwendung und/oder das erfindungsgemäße Verfahren gelten, gelten also *mutatis mutandis* auch für die erfindungsgemäße Zusammensetzung (Wirkstoffzusammensetzung) und umgekehrt.

Die Wirkstoffzusammenzusammensetzung kann frei von Komponente (a2) sein, es ist aber bevorzugt, dass die Wirkstoffzusammenzusammensetzung die Komponente (a2) enthält.

Es ist bevorzugt, dass die Wirkstoffzusammensetzung (im Wesentlichen) aus den Komponenten (a1) und (b), besonders bevorzugt aus den Komponenten (a1), (a2) und (b) besteht.

Es ist weiterhin bevorzugt, dass der Massenanteil des Trägers bezogen auf die Gesamtmasse der Wirkstoffzusammensetzung 40 % bis 99 %, vorzugsweise 70 % bis 99 %, insbesondere 80 % bis 99 % beträgt.

Es ist weiterhin bevorzugt, dass der Massenanteil des mindestens einen mikrobiologischen Wirkstoffs bezogen auf die Gesamtmasse der Wirkstoffzusammensetzung 1 % bis 60 %, vorzugsweise 1 % bis 30 %, insbesondere 1 % bis 20 % beträgt.

Es ist daher bevorzugt, dass der Massenanteil des Trägers bezogen auf die Gesamtmasse der Wirkstoffzusammensetzung 40 % bis 99 %, vorzugsweise 70 % bis 99 %, insbesondere 80 % bis 99 % beträgt und dass der Massenanteil des mindestens einen mikrobiologischen Wirkstoffs bezogen auf die Gesamtmasse der Wirkstoffzusammensetzung 1% bis 60 %, vorzugsweise 1 % bis 30 %, insbesondere 1 % bis 20 % beträgt.

Es ist weiterhin bevorzugt, dass das Verhältnis der Masse der Trägerzusammensetzung zur Masse des mindestens einen mikrobiologischen Wirkstoffs von 1:1 bis 20:1, vorzugweise von 3:1 bis 15:1, insbesondere von 5:1 bis 10:1 beträgt.

Erfindungsgemäß beträgt der $a_w$-Wert der Wirkstoffzusammensetzung ebenfalls weniger als 0,4, vorzugsweise weniger als 0,3, insbesondere weniger als 0,25. Der $a_w$-Wert wird dabei vorzugsweise wie für den Träger bestimmt.

Es ist weiterhin bevorzugt, dass die Wirkstoffzusammensetzung flüssig ist, beispielsweise also als Öldispersion (OD), Dispersionskonzentrat (DC) oder Suspensionskonzentrat (SC) vorliegt. Dies hat den Vorteil, dass die Zusammensetzung einfach zu handhaben ist. Es ist aber auch möglich, dass die Wirkstoffzusammensetzung fest ist, sie also beispielsweise als ein in Wasser dispergierbares Pulver (WP) oder als ein in Wasser dispergierbares Granulat (WG) vorliegt.

Die Wirkstoffzusammensetzung ist erhältlich, indem man den mikrobiologischen Wirkstoff mit dem Träger mischt. Es ist bevorzugt, dass der mikrobiologische Wirkstoff im Träger gelöst und/oder suspendiert und/oder dispergiert wird. Der mikrobiologische Wirkstoff wird dabei zuvor vorzugsweise auf einem dafür geeigneten Nährboden nach an sich bekannten Methoden, wie zum Beispiel der Submersfermentation oder der Feststofffermentation, kultiviert. Vorzugsweise wird der kultivierte Mikroorganismus durch geeignete Separations-, Trocknungs-, Mahl- und/oder Dispergierverfahren aufbereitet. Dabei werden vorzugsweise im Anschluss an die Kultivierung der Mikroorganismus und/oder seine bevorzugt verwendeten Organe vom Kultursubstrat vorzugsweise separiert. In einer besonders bevorzugten Variante wird das von dem Mikroorganismus bewachsene Kultursubstrat (insbesondere im Falle der Verwendung von festen Kultursubstraten) zuvor getrocknet. In einer anderen Variante kann der Mikroorganismus bzw. seine bevorzugt verwendeten Organe nach seiner Separation vom Kultursubstrat beispielsweise mit Hilfe von Gefrier- oder Sprühtrocknungsmethoden getrocknet werden. Nach der Separation sowie gegebenenfalls Trocknung werden der Mikroorganismus und/oder seine Organe im Träger suspendiert und/oder dispergiert. Es ist weiterhin bevorzugt, dass man den Mikroorganismus, vorzugsweise ausgewählt aus der Gruppe der Pilze, durch Mahl- und/oder Dispergierverfahren aufbereitet. Dabei erfolgt nach der Kultivierung, vor der Separation des Mikroorganismus und/oder seiner bevorzugt verwendeten Organe eine Aufbereitung des bewachsenen Kultursubstrates durch ein geeignetes Dispergierverfahren oder nach der Trocknung durch ein geeignetes Mahlverfahren. Vorzugsweise erfolgt dann anschließend eine Separation/Isolation des Mikroorganismus bzw. seiner bevorzugt verwendeten Organe durch an sich bekannte Verfahren, wie Sieb-, Filtrations-, Windsicht-, Dekantier- und/oder Zentrifugationsverfahren. Vorzugsweise erfolgt die Herstellung der Wirkstoffzusammensetzung durch Einmischen des mindestens einen Mikroorganismus und/oder seiner Organe in den Träger, vorzugsweise in einem Mischkessel unter Verwendung eines Rührers. Dabei erhält man vorzugsweise eine flüssige Wirkstoffzusammensetzung, wie beispielsweise eine Öldispersion (OD), Suspensionskonzentrat (SC) oder eines Dispersionskonzentrats (DC). Durch Auswahl geeigneter Polyglycerinester und/oder Verwendung entsprechender Viskositätsregler kann die Viskosität dabei so eingestellt werden, dass keine oder zumindest nur eine verminderte Separation der eingemischten Mikroorganismen in der flüssigen Formulierung, vorzugsweise einer SC- und DC-Formulierung, zu beobachten ist.

Die Wirkstoffzusammensetzung wird für die Anwendung auf Pflanzen bzw. dem Boden vorzugsweise im Sprühtank mit Wasser zu einer Spritzbrühe verdünnt. Bevorzugt beträgt der Massenanteil des Wassers bezogen auf die Gesamtmasse der Spritzbrühe 80 % bis 99,99 %, vorzugsweise 90 % bis 99,9 %, insbesondere 95 % bis 99 %. Der Massenanteil kann aber auch darüber oder darunter liegen, je nach Aufwandmenge des mikrobiologischen Wirkstoffs. Es ist bevorzugt, die Spritzbrühe mit maximal 1000 Litern, vorzugsweise 50 Litern bis 600 Litern, insbesondere mit 100 Litern bis 400 Litern Wasser pro Hektar zu versprühen, was sich nach der Aufwandmenge des mikrobiologischen Wirkstoffs und nach Art und Anzahl der Pflanzen richtet.

Die Wirkstoffzusammensetzung zeigt bei Verdünnung der Zusammensetzung im Sprühtank eine hohe Keimrate. Es ist daher weiterhin bevorzugt, dass die Keimrate, bestimmt wie in den Beispielen beschrieben, mindestens 50 %, vorzugsweise mindestens 70 %, insbesondere mindestens 90% bezogen auf den Startwert beträgt.

Ein weiterer Gegenstand der vorliegenden Erfindung ist die Verwendung der erfindungsgemäßen Wirkstoffzusammensetzung für die Behandlung von Pflanzen und/oder von Saatgut und/oder von Böden und/oder die Verwendung als Biostimulanz.

Vorzugsweise wird die erfindungsgemäße Wirkstoffzusammensetzung als biologisches Pflanzenschutzmittel, biologisches Pflanzenstärkungsmittel oder biologisches Bodenverbesserungsmittel eingesetzt, besonders bevorzugt wird die erfindungsgemäße Wirkstoffzusammensetzung für den Pflanzenschutz eingesetzt.

Bei der Verwendung für den Pflanzenschutz, für die Behandlung von Saatgut und/oder als Biostimulanz wird die Wirkstoffzusammensetzung vorzugsweise in den Boden eingemischt oder eingewässert oder auf/an der Pflanze oder auf/an dem Saatgut appliziert. Dabei wird die Wirkstoffzusammensetzung gegebenenfalls je nach beabsichtigtem Anwendungszweck mit Wasser auf die Anwendungskonzentration verdünnt.

Bevorzugt werden die erfindungsgemäßen Wirkstoffzusammensetzungen als Formulierung, vorzugweise als Pflanzenschutzformulierung, für Spritzbrühen verwendet. Bevorzugt beträgt dabei der Massenanteil des Trägers bezogen auf die Gesamtmasse der Spritzbrühe von 0,001 % bis 1 %, weiter bevorzugt von 0,01 % bis 0,5 %.

Bevorzugt wird die Spritzbrühe über ein Bewässerungssystem, ausgewählt aus der Gruppe bestehend aus Mikrobewässerungssystemen, Sprinklersystemen und Drip-Systemen, auf/an die Pflanze gebracht.

Pflanzenschutzformulierungen werden für ihre Anwendung auf Pflanzen oder Pflanzenteile meist vor dem üblichen Aussprühen über Düsen mit Wasser verdünnt und enthalten neben der wirksamen Komponente auch andere Hilfsmittel, wie Emulgatoren, Dispergierhilfsstoffe, Antifrostmittel, Entschäumer, Biozide und oberflächenaktive Substanzen wie Tenside. Aktivstoffe, insbesondere Fungizide, Insektizide und Nährstoffe können auch allein oder in Kombination und versehen mit oben angegebenen anderen Hilfsmitteln mit verschiedenen Methoden auf Samen (Saatgut) von Pflanzen aufgebracht werden. Solche Methoden werden auch Saatgutbehandlungsmethoden genannt. Die Saatgutbehandlung mit Fungiziden und Insektiziden kann Pflanzen im frühen Stadium des Wachstums vor Krankheiten und Insektenbefall schützen.

Die Pflanzenschutzformulierungen können auch mittels Pflanzen bestäubende Insekten, sogenannte "Bestäuber", wie beispielsweise Hummeln oder Bienen, auf die Pflanzen aufgebracht werden. Dabei wird die Zusammensetzung gegebenenfalls mit Wasser auf die Anwendungskonzentration verdünnt. Vorzugsweise wird die Zusammensetzung aber unverdünnt eingesetzt. Die Verbreitung von chemischen Pflanzenschutzmitteln mittels bestäubenden Insekten ist beispielsweise in WO 2011026983 A1 beschrieben. Auf entsprechende Weise lassen sich auch biologische Pflanzenschutzmittel verbreiten. Dabei ist es vorteilhaft, wenn die Bestäuber nicht durch den mikrobiologischen Wirkstoff bzw. die Zusammensetzung beeinträchtigt oder geschädigt werden.

Kommen Biozide in den Formulierungen zur Anwendung, so werden sie so ausgewählt, dass sie die Mikroorganismen der erfindungsgemäßen Zusammensetzungen nicht schädigen. Dies bedeutet, dass die Mikroorganismen in der Formulierung nur wenig bis gar nicht in ihrer Lebensfähigkeit und/oder Keimfähigkeit eingeschränkt werden. Bevorzugt bleibt die Lebensfähigkeit und/oder Keimfähigkeit 2 Stunden nach Ansetzen der Formulierung zu mindestens 80%, bevorzugt mindestens 90%, mehr bevorzugt zu mindestens 95% erhalten. Die Untersuchung erfolgt wie in den Beispielen beschrieben.

Eine Wirkstoffzusammensetzung enthaltend Konidien von *Paecilomyces lilacinus* als mikrobiellen Wirkstoff kann für die biologische Bekämpfung von pflanzenparasitären Nematoden eingesetzt werden. Bei Verwendung der Sporen von *Talaromyces flavus* kann das Präparat zur Bekämpfung von *Verticillium dahliae,* einem Krankheitserreger, der an Baumwolle eine wirtschaftlich relevante Welke hervorruft, verwendet werden. Zusammensetzungen, die Sporen von *Metarhizium rileyi (Nomuraea rileyi)* enthalten, können zur Bekämpfung der Raupen verschiedener schädlicher Schmetterlingsarten, wie beispielsweise *Helicoverpa armigera* und *Spodoptera exigua,* eingesetzt werden. Die Anwendung der Zusammensetzung unter Verwendung der Konidien von *Penicillium bilaii* erhöht die Verfügbarkeit von mineralischem Phosphor im Boden.

Bevorzugte landwirtschaftliche Einsatzgebiete der Wirkstoffzusammensetzungen sind der Ackerbau, der Garten- und Zierpflanzenbau, der Weinbau und der Baumwollanbau. Besonders bevorzugt ist der Obst- und Gemüsebau. Bevorzugtes Obst ist Kernobst, Steinobst, Beerenobst und Schalenobst. Bevorzugtes Gemüse ist Wurzelgemüse, Sprossgemüse, Knollengemüse, Zwiebelgemüse, Blattstielgemüse, Blattgemüse, Blattsalate, Samengemüse, Fruchtgemüse.

Im Falle der Verwendung der Wirkstoffzusammensetzung

i) für die Behandlung von Pflanzen; oder
ii) für die Behandlung von Saatgut; oder
iii) für die Behandlung von Böden; oder
iv) als Biostimulanz;

wird die Wirkstoffzusammensetzung vorzugsweise als Formulierung für Spritzbrühen eingesetzt, wobei der Massenanteil der Trägerzusammensetzung bezogen auf die Gesamtmasse der Spritzbrühe 0,001% bis 1% beträgt.

Ein weiterer Gegenstand der vorliegenden Erfindung ist die Verwendung der Wirkstoffzusammensetzung als probiotisches Nahrungsergänzungsmittel und/oder probiotisches Futtermitteladditiv. Die Wirkstoffzusammensetzungen können als Probiotikum in Nahrungs- und/oder Futtermitteln verwendet werden. Probiotische Nahrungs- und/oder

Futtermittel enthalten als mikrobiellen Wirkstoff üblicherweise Bakterien und/oder Pilze. Zu den probiotischen Nahrungsmitteln zählen beispielsweise Joghurtzubereitungen, Kefirzubereitungen, Sauermilchzubereitungen sowie milchsauer vergorenes Gemüse. Der mikrobielle Wirkstoff entfaltet dabei im Darm eine gesundheitsfördernde Wirkung.

Ein weiterer Gegenstand der vorliegenden Erfindung ist eine erfindungsgemäße Wirkstoffzusammensetzung zur Verwendung als probiotisches Arzneimittel.

Die Trägerzusammensetzungen bzw. die Wirkstoffzusammensetzungen haben zahlreiche Vorteile:

Ein Vorteil ist die Verbesserung der Lagerfähigkeit von Mikroorganismen durch Verwendung der Trägerzusammensetzung bzw. eine verbesserte Lagerfähigkeit der Wirkstoffzusammensetzung. Insbesondere kann die Wirkstoffzusammensetzung viele Wochen bei Raumtemperatur gelagert werden. Dies vereinfacht den Transport und die Lagerung. Die Lagerung und der Transport der Zusammensetzung erfolgen dabei vorzugsweise unter Luftabschluss in luftdicht verschlossenen Flaschen, Beuteln, Kanistern oder Fässern. Die erhöhte Lagerfähigkeit führt insbesondere zu einer Erhöhung der biologischen Aktivität.

Des Weiteren zeigt die Wirkstoffzusammensetzung, insbesondere in Form eines Dispersions- bzw. Suspensionskonzentrats, eine verbesserte Lebensfähigkeit und/oder Keimfähigkeit gegenüber dem Stand der Technik.

Eine weitere Verbesserung gegenüber dem Stand der Technik ist, dass die Mikroorganismen und/oder ihre Organe in den anwendungsfertigen wässrigen Verdünnungen deutlich länger lebensfähig und/oder keimfähig bleiben als in den wässrigen Verdünnungen basierend auf dem Stand der Technik.

Formulierungen von Pilzsporen können beispielsweise vor der Anwendung in einer Vormischung mit Wasser angesetzt werden, um die Keimung zu beschleunigen und die Infektionszeit zu verringern (vgl. H.D. Burges: Formulation of Microbial Biopesticides, Springer, 1998). Ebenso empfehlen manche Hersteller mikrobieller Produkte (z.B. Remedier® von Isagro, Naturalis® von CBC Europe, FZB24 von ABiTEP GmbH), die Sporen in der Formulierung vor dem Sprühauftrag zu aktivieren. Dazu wird die Formulierung in einem kleineren Volumen Wasser in einem Gefäß verdünnt (Faktor 3 - 50) und diese 2 bis 24 Stunden vor dem Sprühen stehen gelassen. Da die Mikroorganismen in dieser Phase besonders empfindlich sind, ist es empfehlenswert, in der Formulierung eine biokompatible Trägerzusammensetzung ohne nachteilige Auswirkungen auf den Mikroorganismus zu verwenden. Die erfindungsgemäße Wirkstoffzusammensetzung zeichnet sich nun durch eine höhere Überlebensfähigkeit der enthaltenen Mikroorganismen bei Raumtemperatur bzw. leicht erhöhten Temperaturen aus. Somit ist sie unkompliziert zu lagern und zu transportieren und benötigt keine Kühlung, um zu gewährleisten, dass eine ausreichend hohe Konzentration keim- bzw. lebensfähiger Mikroorganismen den Zielort auf der Pflanze oder im Boden erreicht. In den anwendungsfertigen wässrigen Verdünnungen beeinträchtigen die erfindungsgemäßen Trägerzusammensetzungen bzw. Wirkstoffzusammensetzungen nicht das Auskeimen bzw. das Wachstum der Mikroorganismen am Zielort.

Ein weiterer Vorteil ist die biologische Abbaubarkeit des Polyglycerinesters, des Trägers und der Zusammensetzung umfassend den Träger und den mikrobiologischen Wirkstoff. Die biologische Abbaubarkeit wird dabei vorzugsweise bestimmt nach der OECD 301 F Methode bestimmt. Weiter bevorzugt wird die biologische Abbaubarkeit nach OECD 301 F nach 28 Tagen bei 22 °C bestimmt.

Ein weiterer Vorteil ist, dass die Adhäsion und Retention von Sprays/Spritzbrühen, die die Trägerzusammensetzung bzw. die Wirkstoffzusammensetzung enthalten, auch auf schwer zu benetzenden Pflanzenoberflächen verbessert wird.

Ein weiterer Vorteil ist, dass die Wirkstoffaufnahme durch die Cuticula und die Vakuolen der Epidermiszellen der Pflanze aktiviert werden. Dies führt dazu, dass die Menge an verwendetem Pflanzenschutzmittel zu Bewirtschaftung reduziert werden kann, was sowohl ökologische als auch ökonomische Vorteile hat.

Ein weiterer Vorteil ist auch die sehr gute Regenfestigkeit der Formulierung auf den Pflanzen nachdem das Wasser verdunstet ist. Auch nach einem Regen ist der mikrobiologische Wirkstoff auf den Blättern vorhanden. Somit ist die biologische Wirksamkeit auch nach einem Regen gewährleistet.

Ein weiterer Vorteil ist die Ertragssteigerung. So zeigen Freilandversuche, dass sowohl die Trägerzusammensetzung alleine als auch in Kombination mit dem mikrobiologischen Wirkstoff eine ertragssteigernde Wirkung auf landwirtschaftliche Nutzpflanzen haben. Ein weiterer Vorteil ist daher insbesondere auch die Steigerung der Effektivität von biologischen Pestiziden. Die Ertragssteigerung landwirtschaftlicher Nutzpflanzen wie auch die Wirkungsverstärkung der Pestizide ist für eine Vielzahl von Kulturpflanzen erfolgreich. So ist dies sowohl bei einkeimblättrigen als auch bei zweikeimblättrigen Pflanzen zu beobachten. Da sich die wirkungsverstärkenden Effekte bei verschiedenen Kulturpflanzen, zweier unterschiedlicher Gruppen (die Verwendung des Begriffs "Gruppe" ist im botanischen Sinne zu verstehen) der Bedecktsamer, sowohl einkeimblättrig als auch zweikeimblättrig, zeigen, kann davon ausgegangen werden, dass die Effekte auch mit anderen Pflanzen möglich sein werden.

Ein weiterer Vorteil ist, dass der Träger als auch die Wirkstoffzusammensetzung Anti-Drift-Eigenschaften aufweisen. Die Verringerung der Driftfähigkeit der Spraytröpfchen ist vorteilhaft, da so eine Verringerung der Kontamination der Umwelt bewirkt wird. Darüber hinaus kann der Verlust von teuren Wirkstoffen vermieden werden, da diese zu einem höheren Prozentsatz auf der Target-Fläche ausgebracht werden können. Unter "Drift" wird im Umfang der Erfindung die transversale Fortbewegung eines Sprays von seinem Entstehungsort verstanden. Drift wird üblicherweise

durch Umwelt- oder/und Umgebungseinflüsse verursacht, wie beispielsweise Wind. Dieser Wind kann natürlichen oder künstlichen Ursprungs sein. Wind künstlichen Ursprungs ist beispielsweise eine Luftströmung, die durch die Fortbewegung eines Fahrzeuges zu Lande oder eines Flugzeuges in der Luft entstehen. Das Spray ist hierbei in allen Fällen ein wässriges Medium. Bevorzugt entsteht das Spray durch Zerstäubung in der Luft. Bevorzugt wird unter Drift die transversale Fortbewegung eines Sprays vom Ort der Entstehung durch Wind verstanden, wobei das Spray durch Zerstäubung eines wässrigen Mediums in der Luft entstanden ist. Die Anti-Drift-Eigenschaften lassen sich bevorzugt durch den Einfluss der Trägerzusammensetzung bzw. der Wirkstoffzusammensetzung auf die Tröpfchengrößenverteilung des Sprays quantifizieren. Es besteht eine direkte Beziehung zwischen der Größe eines Tröpfchens und seiner Driftneigung - je feiner das Tröpfchen, umso größer die Driftgefahr. Der Begriff "Tröpfchengrößenverteilung" bezieht sich dabei auf volumengewichtete Größenverteilungen bei einer Messung der Durchmesser der Tröpfchen im Sprühnebel. Die Tröpfchengrößenverteilung eines Sprays ist von der Zusammensetzung des Sprays sowie von den Bedingungen während des Sprühvorgangs abhängig. So haben beispielsweise die Bauart der verwendeten Spritzdüse sowie der gewählte Spritzdruck einen signifikanten Einfluss auf die resultierende Tröpfchengrößenverteilung. Bevorzugt wird das Spray durch Anwendung von Düsen erzeugt, bevorzugt von Düsen der Bauarten Flachstrahldüsen, Weitwurfflachstrahldüsen, Doppelflachstrahldüsen, Hohlkegeldüsen, Vollkegeldüsen, Hochdruckdüsen, Randdüsen sowie Air-Injektordüsen, besonders bevorzugt sind Düsen der Bauart einer Flachstrahldüse. Derartige Düsen sind zum Beispiel von den Herstellern Lechler, TeeJet und/oder Agrotop erhältlich. Insbesondere bevorzugt sind Flachstrahldüsen der Firma TeeJet, wobei Düsen des Typs XR 11003 ganz besonders bevorzugt sind. Weiterhin bevorzugt wird ein Druck von 0,5 bis 10 bar, bevorzugt von 0,8 bis 8 bar, mehr bevorzugt von 0,9 bis 6 bar, weiter mehr bevorzugt von 0,95 bis 2,5 bar und insbesondere bevorzugt von 1 bis 1,5 bar zur Sprayerzeugung angewendet. Der Einfluss der Trägerzusammensetzung bzw. der Wirkstoffzusammensetzung ist immer relativ bezogen auf ein Spray einer Formulierung, welche sich durch die Abwesenheit der Trägerzusammensetzung bzw. der Wirkstoffzusammensetzung auszeichnet und unter identischen Bedingungen versprüht wird. Dabei lässt sich eine relative Verschiebung des volumenbezogenen Maximums und/oder des volumenbezogenen Medians der Tröpfchengrößenverteilung von mindestens 5 %, vorzugsweise mindestens 10 %, insbesondere mindestens 15 %, bezogen auf die Tröpfchengrößenverteilung eines identischen Sprays ohne Trägers bzw. ohne eine Zusammensetzung aus Träger und mikrobiologischen Wirkstoff beobachten.

Ein weiter Vorteil ist, dass der Träger die Dispergierung des mikrobiologischen Wirkstoffs in einer wässrigen Zusammensetzung, wie beispielsweise der Spritzbrühe erleichtert.

Ein weiterer Vorteil ist, dass der Träger selbstemulgierend eingestellt werden kann. Die Trägerzusammensetzung sowie die Wirkstoffzusammensetzung lassen sich leicht in Wasser dispergieren. Unter "selbstemulgierend" wird verstanden, dass sich der Träger bzw. die Wirkstoffzusammensetzung ohne großen Schereintrag in Wasser dispergieren lässt und sich hierbei spontan Emulsionströpfchen mit einer mittleren Größe von weniger als 400 μm, vorzugsweise weniger als 200 μm, insbesondere weniger 100 μm bilden. Die Größe der Emulsionströpfchen kann beispielsweise durch Laserbeugung bestimmt werden beispielsweise durch Verwendung von Laserbeugungssystemen oder durch die computergestützte Bildauswertung von hochaufgelösten, statischen Aufnahmen des Sprühnebels. Die Größe der Emulsionströpfchen wird bevorzugt durch Laserbeugung gemessen, besonders bevorzugt durch Verwendung des MasterSizers 3000 der Firma Malvern. Da Effizienzverstärker für Pflanzenschutzmittel in der Regel wasserlöslich sind, um so die Wirksamkeit von Pflanzenschutzmitteln aus wässrigen Spritzbrühen heraus zu verbessern, ist es im Lichte des Stands der Technik überraschend, dass ähnliche Effekte auch mit selbstemulgierenden Zusammensetzungen erzielt werden können. Der selbstemulgierende Effekt lässt sich insbesondere durch gezielte Einstellung der Hydrophobizität und/oder Wasserlöslichkeit des Polyglycerinesters vorzugsweise in Kombination mit einem geeigneten Emulgator erreichen. Im Fall von Tankmix-Formulierungen kommt es so beispielsweise schon während des Tankmix-Vorgangs zu einer ausreichend homogenen Verteilung des Polyglycerinesters in der Spritzbrühe. Dies erleichtert zum einen die Anfertigung von Spritzbrühen. Des Weiteren kommt es durch die gute Einarbeitbarkeit und der damit verbundenen homogenen Verteilung während des Sprühvorgangs nicht zu einer Verstopfung der Sprühdüsen.

Ein weiterer Vorteil ist, dass Polyglycerinester aus natürlichen Rohstoffen hergestellt werden können. Dies ist im Sinne eines nachhaltigen Wirtschaftens vorteilhaft.

Ein weiterer Vorteil ist, dass Polyglycerinester gesundheitlich unbedenklich sind. In vielen Fällen haben sie sogar eine Zulassung als Lebensmittelzusatz, zum Beispiel unter der Nummer E475 (Polyglycerinester von Speisefettsäuren). All dies ist vorteilhaft

im Hinblick auf die im Stand der Technik beschrieben Rückstandsproblematik.

In den nachfolgend aufgeführten Beispielen wird die vorliegende Erfindung beispielhaft beschrieben. Die Erfindung ist durch die Ansprüche definiert.

## Beispiele

*Trägerzusammensetzung (erfindungsgemäß)*

Als erfindungsgemäße Trägerzusammensetzung wurde BREAK-THRU® SP 133 von Evonik, eine Mischung aus 80 Gew.-% Triglycerintrioleat und 20 Gew.-% Polyethylenglykol-20-Sorbitan-Trioleat, verwendet.

*Bestimmung der Wasseraktivität von Zusammensetzungen*

Zur Bestimmung der Wasseraktivität einer Probe wird die Luftfeuchte nach Erreichen des Feuchtegleichgewichts unmittelbar über einer Probe (Wasserdampf-Partial-Differenzdruck) gemessen. Die Gleichgewichtsfeuchte (equilibrium relative humidity (ERH) wird in % relative Feuchte gemessen und steht mit dem $a_w$-Wert in folgendem Zusammenhang: $a_w$ = ERH/100. Zur Bestimmung der Wasseraktivität der Zusammensetzungen wurde der *LabMaster-aW neo* der Firma Novasina verwendet.

*Herstellung der Zusammensetzungen mit Trichoderma harzianum*

Sporen des Pilzes *Trichoderma harzianum* wurden von der Firma Rhizo-Mic UG bezogen und enthielten laut Elementaranalyse außer den Sporen ungefähr 75 Gew.-% $SiO_2$. Das Pulver enthielt 1,97 x $10^9$ keimfähige Sporen/g Produkt. Die erfindungsgemäße Wirkstoffzusammensetzung aus BREAK-THRU® SP 133 und Sporen von *Trichoderma harzianum* wurde wie folgt hergestellt: 3,60 g des Sporen enthaltenden Pulvers wurden in ein 50 mL Zentrifugenröhrchen (z.B. sterile 50 mL Röhrchen von Greiner Bio-One GmbH) eingewogen und mit 26,40 g BREAK-THRU® SP 133 überschichtet. Die Mischung wurde für 30 Sekunden auf einem Vortexschüttler (*lab dancer* der Firma ika) gemischt. Nach Homogenisieren mit einem Spatel wurde die Zusammensetzung nach einer Wartezeit von 15 Minuten erneut für 30 Sekunden auf einem Vortexschüttler gemischt. Die hergestellte Zusammensetzung enthielt 1,95 x $10^8$ keimfähige Sporen/g. Die Zusammensetzungen der Vergleichsbeispiele wurden analog hergestellt. Für die Vergleichsbeispiele wurden Glyzerin (reinst, min. 98%, wasserfrei; Bernd Kraft GmbH), PEG 400 (Kollisolv® PEG E 400; Sigma Aldrich, mittlere Molmasse 320-420 g/mol), Pluronic® PE 6400 (BASF) und Sonnenblumenöl (Lebensmittelqualität) als flüssige Carrier verwendet. Die kommerzielle WP-Formulierung von *Trichoderma harzianum* war Trianum® P (Koppert).

*Bestimmung der Lagerstabilität*

Die hergestellten Zusammensetzungen mit Sporen von *Trichoderma harzianum* wurden vier Wochen bei 40°C inkubiert und die Anzahl koloniebildender Einheiten (KBE) unmittelbar nach der Herstellung (Startwert) sowie nach 7, 14, 21 und 28 Tagen bestimmt. Die Anzahl kolonie-bildender Einheiten (KBE) ist ein Maß für die Anzahl der Sporen, die vor bzw. nach der Lagerung in der Lage waren, auszukeimen und Kolonien zu bilden. Zur Bestimmung der Anzahl koloniebildender Einheiten (KBE) nach dem Plattenverfahren wurde 1,0 g des Probenmaterials mit steriler physiologischer Kochsalzlösung (0,9 Gew.-% NaCl in Wasser) in einer dezimalen Verdünnungsreihe bis zur Stufe $10^{-8}$ verdünnt. Es wurden die drei Verdünnungsstufen $10^{-6}$, $10^{-7}$ und $10^{-8}$ (jeweils 1,0 mL) auf Fertignährmedium (Compact Dry YM für Hefen und Schimmelpilze oder Compact Dry Total Count von Nissui Pharmaceutical Co., Ltd.) ausplattiert. Die Pilzsporen wurden drei Tage bei 25°C bebrütet. Ausgewertet wurden solche Platten auf denen 10-100 KBE sichtbar sind. Tabelle 1 zeigt den prozentualen Anteil koloniebildender Einheiten (in KBE/g) bezogen auf den Startwert, als Maß für die Überlebensrate bzw. für die Lagerstabilität der Zusammensetzung. Die dargestellten Ergebnisse sind arithmetische Mittelwerte einer Dreifachbestimmung.

Tabelle 1: Lagerstabilitätsversuche

| Zusammensetzung | | $a_w$-Wert | Anteil keimfähiger Sporen nach Lagerung bei 40°C:[3)] | | |
|---|---|---|---|---|---|
| | | Nach 7 Tagen | Nach 14 Tagen | Nach 21 Tagen | Nach 28 Tagen |
| BREAK-THRU® SP133 (88 Gew.-%) + *T. harzianum* Sporen (12 Gew.-%) [1)] | 0,22 | 21% | 19% | 20% | 12% |
| *T. harzianum* Sporen [2)] | 0,33 | 73% | 30% | 7% | 2% |
| Glyzerin (80 Gew.-%) + *T. harzianum* Sporen (20 Gew.-%) [2)] | 0,10 | 2% | 0,2% | n.b. [4)] | n.b.[4)] |
| PEG 400 (80 Gew.-%) + *T. harzianum* Sporen (20 Gew.-%) [2)] | 0,05 | n.b.[4)] | 21% | 9% | 2% |

(fortgesetzt)

| Zusammensetzung | | $a_w$-Wert | Anteil keimfähiger Sporen nach Lagerung bei 40°C:[3] | | |
|---|---|---|---|---|---|
| | | Nach 7 Tagen | Nach 14 Tagen | Nach 21 Tagen | Nach 28 Tagen |
| Pluronic® PE 6400 (80 Gew.-%) + *T. harzianum* Sporen (20 Gew.-%) [2] | 0,20 | 26% | 18% | 8% | 1% |
| Sonnenblumenöl (80 Gew.-%) + *T. harzianum* Sporen (20 Gew.-%) [2] | 0,40 | 50% | 15% | 10% | 0,4% |
| kommerziell erhältliche WP-Formulierung von *T. harzianum* [2] | 0,27 | 19% | 7% | 2% | 2% |
| [1] erfindungsgemäßes Beispiel<br>[2] Vergleichsbeispiel<br>[3] dargestellt als prozentualer Anteil koloniebildender Einheiten (in KBE/g) bezogen auf den Startwert<br>[4] nicht bestimmt | | | | | |

Das erfindungsgemäße Beispiel BREAK-THRU® SP 133 zeigt eine deutlich verbesserte Überlebensrate nach 28 Tagen bei 40°C als die Vergleichsbeispiele inklusive einer kommerziell erhältlichen *Trichoderma Harzianum* WP Formulierung.

*Keimzahlbestimmung in Gegenwart von Adjuvantien*

Durch eine Keimzahlbestimmung in Gegenwart von 1,0 Gew.-% Adjuvantien wurde deren Einfluss auf die Keimfähigkeit bzw. Überlebensfähigkeit verschiedener kommerzieller Mikroorganismen unter anwendungsrelevanten Bedingungen untersucht. Als Vergleichsbeispiele wurden die ebenfalls für den biologischen Anbau zugelassenen Adjuvantien Nu-film®-P (Intrachem Bio Deutschland GmbH) und Wetcit (Oro Agri) verwendet. Die kommerziellen Formulierungen wurden in einer dezimalen Verdünnungsreihe im Verhältnis 1:100.000 bis 1:1.000.000.000 mit steriler physiologischer NaCl-Lösung (0,9 Gew.%) unter Zugabe von 1,0 Gew.% Adjuvant verdünnt und auf einem geeigneten Fertignährmedium (Compact Dry von Nissui Pharmaceutical Co., Ltd.) ausplattiert. Pilzsporen und Hefezellen wurden drei Tage bei 25°C, Bakteriensporen einen Tag bei 30°C bebrütet. Ausgewertet wurden Platten auf denen 10-100 KBE sichtbar sind. Die Keimzahl wurde als arithmetischer Mittelwert einer Dreifachbestimmung bestimmt. Aus der Keimzahl wurde die prozentuale Änderung der Anzahl koloniebildender Einheiten bestimmt, indem die Keimzahl in Gegenwart von BREAK-THRU® SP 133 als Referenz diente und auf 100% gesetzt wurde.

Tabelle 2: Relative Änderung der Anzahl koloniebildender Einheiten verschiedener kommerzieller Mikroorganismen unter Zugabe von Adjuvantien mit BREAK-THRU® SP 133 als Referenz

| Wirkstoff | BREAK-THRU® SP 133[1] | Nu-film®-P [2] | WETCIT® [2] |
|---|---|---|---|
| *Aureobasidium pullulans* (Botector) | 100% | 44% | < 0,01% |
| *Bacillus amyloliquefaciens* (FZB24) | 100% | 97% | < 0,01% |
| *Coniothyrium minitans* (Contans) | 100% | 53% | < 0,01% |
| *Trichoderma harzianum* (Trianum P) | 100% | 74% | < 0,02% |
| [1] erfindungsgemäßes Beispiel<br>[2] Vergleichsbeispiel | | | |

Die Ergebnisse zeigen, dass BREAK-THRU® SP 133 im Vergleich zu anderen Adjuvantien sehr mild ist und das Wachstum verschiedener Mikroorganismen nicht beeinträchtigt.

*Bestimmung der Retention*

Um zu überprüfen ob die erfindungsgemäße Trägerzusammensetzung BREAK-THRU® SP 133 die Fähigkeit besitzt, die Retention von Spritzbrühen an der Kulturpflanze zu verbessern, wurde eine Studie am *Plant Protection Chemistry Institut in Neuseeland (PPCNZ)* durchgeführt. Als Modellpflanze diente Spinat (*Spinacia oleracea var.*

*Perpetual*). Basierend auf Kontaktwinkelmessungen an den adaxialen Blättern der Pflanze gilt Spinat als *"moderately difficult to wet",* also mittelschwer zu benetzen. Zum Vergleich wurde ein polyethermodifiziertes Trisiloxan (BREAK-THRU® S 240, Evonik) als übliches aus dem Stand der Technik bekanntes Adjuvant, herangezogen. Die Spinatpflanzen wurden als 4 Wochen alte Setzlinge von einer Gärtnerei gekauft und in einzelne Töpfe gesetzt. Diese wurden dann in einer Aufzuchtkammer unter kontrollierten Umweltbedingungen (20°C Tag/15°C Nacht, 75% rh, mit einer 12 h andauernden Photoperiode) weiter kultiviert. Zum Zeitpunkt des Versuchs waren die Pflanzen dann 6 Wochen alt und 7 cm groß.

Zur Bestimmung der Retention wurde ein *moving head track sprayer* eingesetzt, der die angesetzten Spritzbrühen auf die Versuchspflanzen sprühte. Der Farbstoff Tartrazin wurde in eine Menge von 8 g/L den verwendeten Spritzbrühen zugesetzt. Jede Variante beinhaltete 25 Wiederholungen/Pflanzen und wurde mit 100 L/ha mittels *Air Induction* Flachstrahldüse (AI 95015EVS) bei 250 kpa Druck und einem Durchfluss von 0,56 L/min behandelt. Dabei wurden die Düsen 50 cm über Pflanzenhöhe (7 cm) befestigt. Um die Applikationsrate zu verifizieren, wurden künstliche Auffänger (Plastikschalen in vierfacher Wiederholung pro Variante) horizontal in Höhe der durchschnittlichen Pflanzenhöhe befestigt.

Nach Ausbringung der Spritzbrühe wurden stichprobenartig drei Blätter von jeder Wiederholung gesammelt und sofort mit entionisiertem Wasser gewaschen. Auch die künstlichen Auffänger wurden sofort nach Applikation mit entionisiertem Wasser gewaschen.

Die der Spritzbrühe hinzugegebene Tartrazin-Farbe wurde nun spektrophotometrisch bei 427 nm quantifiziert. Die Blattfläche wurde mittels Blattflächenmessgerät (Licor LI 3100A) bestimmt. Nun wurde die zurückbleibende Spritzmenge kalkuliert (Tabelle 3). Die Ergebnisse wurden mittels Varianzanalyse und LSD Test (P= 0.05) unter Verwendung des Computerprogramms Statistix berechnet. Wenn nötig wurden die Daten vor der Analyse transformiert.

Tabelle 3: Retentionsversuche

| Additiv | Konzentration % w/v | Retention in $\mu l/cm^2$ | Retention in % |
|---|---|---|---|
| BREAK-THRU® SP 133[1)] | 0,1 | 0,68 a | 68,2 a |
| BREAK-THRU® S 240 [2)] | 0,1 | 0,56 b | 55,6 b |
| [1)] erfindungsgemäßes Beispiel<br>[2)] Vergleichsbeispiel | | | |

Die Ergebnisse in Tabelle 3 dargestellt zeigen, dass die Retention der Spritzbrühe mit dem erfindungsgemäßen Träger BREAK-THRU® SP 133 gegenüber dem Vergleichsbeispiel BREAK-THRU® S 240 auf Spinatblättern signifikant verbessert wird.

## Patentansprüche

1. Verwendung einer Zusammensetzung, umfassend mindestens einen Polyglycerinester und vorzugsweise mindestens einen Emulgator, als Träger für mindestens einen mikrobiologischen Wirkstoff, **dadurch gekennzeichnet, dass** der mindestens eine Emulgator ausgewählt ist aus der Gruppe bestehend aus Sorbitanfettsäureestern und ethoxylierten Sorbitanfettsäureestern, und weiterhin **dadurch gekennzeichnet, dass** der mindestens eine mikrobiologische Wirkstoff ausgewählt ist aus der Gruppe bestehend aus Mikroorganismen, Organen von Mikroorganismen und ihren Mischungen.

2. Verwendung nach Anspruch 1, **dadurch gekennzeichnet, dass** der mindestens eine Polyglycerinester einen HLB-Wert von kleiner oder gleich 8, vorzugsweise von kleiner oder gleich 7, insbesondere von kleiner oder gleich 6,5 aufweist.

3. Verwendung nach Anspruch 1 oder 2, **dadurch gekennzeichnet, dass** die Acyloxyreste des mindestens einen Polyglycerinesters 4 bis 40, vorzugsweise 8 bis 22, insbesondere 10 bis 18 Kohlenstoffatomen aufweisen.

4. Verwendung nach mindestens einem der Ansprüche 1 bis 3, **dadurch gekennzeichnet, dass** der mindestens eine Polyglycerinester eine Verbindung der allgemeinen Formel (I) ist,

$$M_a \, D_b \, T_c \qquad \text{Formel (I);}$$

mit:

$M = [C_3H_5(OR)_2O_{1/2}]$;
$D = [C_3H_5(OR)_1O_{2/2}]$;
$T = [C_3H_5O_{3/2}]$;
a = 1 bis 10, vorzugsweise 2 bis 3, insbesondere 2;
b = 0 bis 10, vorzugsweise größer 0 bis 5, insbesondere 1 bis 3;
c = 0 bis 3, vorzugsweise 0 bis 1, insbesondere 0;
mit der Maßgabe, dass gilt:

a + b + c = 2 bis 20, vorzugsweise 2 bis 4, insbesondere 3;
wobei die Reste R jeweils unabhängig voneinander ausgewählt sind aus der Gruppe bestehend aus Acylresten R'-C(=O)- und H, mit der Maßgabe, dass mindestens ein Rest R ungleich H ist;
wobei die Reste R' jeweils unabhängig voneinander ausgewählt sind aus der Gruppe bestehend aus einbindigen aliphatischen, gesättigten oder ungesättigten Kohlenwasserstoffresten mit 3 bis 39, vorzugsweise 7 bis 21, insbesondere mit 9 bis 17 Kohlenstoffatomen.

**5.** Verwendung nach mindestens einem der Ansprüche 1 bis 4, **dadurch gekennzeichnet, dass** der mindestens eine Emulgator einen HLB-Wert von größer oder gleich 9, vorzugsweise von größer oder gleich 10, insbesondere von größer oder gleich 11 aufweist.

**6.** Verwendung nach mindestens einem der Ansprüche 1 bis 5, **dadurch gekennzeichnet, dass** der mindestens eine Emulgator ausgewählt ist aus ethoxylierten Sorbitanfettsäureestern.

**7.** Verwendung nach mindestens einem der Ansprüche 1 bis 6, **dadurch gekennzeichnet, dass** die Acyloxyreste des mindestens einen Sorbitanfettsäureesters beziehungsweise ethoxylierten Sorbitanfettsäureesters 4 bis 40, vorzugsweise 8 bis 22, insbesondere 10 bis 18 Kohlenstoffatome aufweisen und/oder dass der mindestens eine Sorbitanfettsäureester beziehungsweise ethoxylierte Sorbitanfettsäureester 0 bis 40, vorzugsweise 10 bis 30, insbesondere 15 bis 25 Oxyethlyen-Gruppen aufweist.

**8.** Verwendung nach mindestens einem der Ansprüche 1 bis 7, **dadurch gekennzeichnet, dass** der mindestens eine Polyglycerinester Triglycerintrioleat und/oder der mindestens eine Emulgator Polyethylenglykol-20-Sorbitan-Trioleat ist.

**9.** Verwendung nach mindestens einem der Ansprüche 1 bis 8, **dadurch gekennzeichnet, dass** der Massenanteil des mindestens einen Polyglycerinesters bezogen auf die Gesamtmasse der Zusammensetzung 60 % bis 100 %, vorzugsweise 70 % bis 90 %, insbesondere 75 % bis 85 % beträgt; und der Massenanteil des mindestens einen Emulgators bezogen auf die Gesamtmasse der Zusammensetzung 0 % bis 40 %, vorzugsweise 10 % bis 30 %, insbesondere 15 % bis 25 % beträgt.

**10.** Verwendung nach mindestens einem der Ansprüche 1 bis 9, **dadurch gekennzeichnet, dass** der mindestens eine mikrobiologische Wirkstoff ausgewählt ist aus der Gruppe bestehend aus *Trichoderma harzianum, Bacillus amyloliquefaciens, Beauveria bassiana, Metarhizium rileyi, Metarhizium anisopliae, Clonostachys rosea, Aureobasidium pullulans, Coniothyrium minitans* und ihren Organen, wobei die Organe vorzugsweise ausgewählt sind aus der Gruppe bestehend aus Sporen, Konidien, Blastosporen, Chlamydosporen, Sklerotien und Hyphensegmenten.

**11.** Verwendung nach mindestens einem der Ansprüche 1 bis 10, **dadurch gekennzeichnet, dass** der mindestens eine mikrobiologische Wirkstoff Sporen, vorzugsweise Pilzsporen und/oder Bakteriensporen, insbesondere Sporen von *Trichoderma harzianum* und/oder von *Bacillus amyloliquefaciens* und/oder von *Beauveria bassiana* und/oder von *Metarhizium rileyi* und/oder von *Metarhizium anisopliae* und/oder von *Clonostachys rosea* und/oder von *Aureobasidium pullulans* und/oder von *Coniothyrium minitans* umfasst.

**12.** Verwendung nach mindestens einem der Ansprüche 1 bis 11, **dadurch gekennzeichnet, dass** der mindestens eine mikrobiologische Wirkstoff Sporen von *Trichoderma harzianum* umfasst oder aus ihnen besteht.

**13.** Verwendung nach mindestens einem der Ansprüche 1 bis 12, **dadurch gekennzeichnet, dass** der $a_w$-Wert der Zusammensetzung weniger als 0,4, vorzugsweise weniger als 0,3, insbesondere weniger als 0,25 beträgt.

**14.** Verwendung nach mindestens einem der Ansprüche 1 bis 13 zur Steigerung der Lagerstabilität des mikrobiologischen Wirkstoffs.

**15.** Verfahren zur Steigerung der Lagerstabilität mindestens eines mikrobiologischen Wirkstoffs durch Verwendung einer Zusammensetzung, umfassend mindestens einen Polyglycerinester und vorzugsweise mindestens einen Emulgator, als Träger für mindestens einen mikrobiologischen Wirkstoff, gemäß den Vorgaben nach mindestens einem der Ansprüche 1 bis 13.

**16.** Zusammensetzung, umfassend:

(a) einen Träger, umfassend:

(a1) mindestens einen Polyglycerinester und vorzugsweise
(a2) mindestens einen Emulgator ausgewählt aus der Gruppe bestehend aus Sorbitanfettsäureestern und ethoxylierten Sorbitanfettsäureestern,

und
(b) mindestens einen mikrobiologischen Wirkstoff;

gemäß den Vorgaben nach mindestens einem der Ansprüche 1 bis 13, **dadurch gekennzeichnet, dass** der $a_w$-Wert der Wirkstoffzusammensetzung weniger als 0,4, vorzugsweise weniger als 0,3, insbesondere weniger als 0,25 beträgt.

**17.** Verwendung der Zusammensetzung nach Anspruch 16

i) für die Behandlung von Pflanzen; oder
ii) für die Behandlung von Saatgut; oder
iii) für die Behandlung von Böden; oder
iv) als Biostimulanz; oder
v) als probiotisches Nahrungsergänzungsmittel und/oder probiotisches Futtermitteladditiv.

**18.** Zusammensetzung nach Anspruch 16 zur Verwendung als probiotisches Arzneimittel.

**Claims**

**1.** Use of a composition comprising at least one polyglycerol ester and preferably at least one emulsifier as carrier for at least one active microbiological ingredient, **characterized in that** the at least one emulsifier is selected from the group consisting of sorbitan fatty acid esters and ethoxylated sorbitan fatty acid esters, and further **characterized in that** the at least one active microbiological ingredient is selected from the group consisting of microorganisms, organs of microorganisms and mixtures thereof.

**2.** Use according to Claim 1, **characterized in that** the at least one polyglycerol ester has an HLB value of not more than 8, preferably of not more than 7, especially of not more than 6.5.

**3.** Use according to Claim 1 or 2, **characterized in that** the acyloxy radicals of the at least one polyglycerol ester have 4 to 40, preferably 8 to 22 and especially 10 to 18 carbon atoms.

**4.** Use according to at least one of Claims 1 to 3, **characterized in that** the at least one polyglycerol ester is a compound of the general formula (I), $M_aD_bT_c$ Formula (I);
with:

$M = [C_3H_5(OR)_2O_{1/2}]$;
$D = [C_3H_5(OR)_1O_{2/2}]$;
$T = [C_3H_5O_{3/2}]$;
a = 1 to 10, preferably 2 to 3, especially 2;
b = 0 to 10, preferably greater than 0 to 5, especially 1 to 3;
c = 0 to 3, preferably 0 to 1, especially 0;
with the proviso that:

a + b + c = 2 to 20, preferably 2 to 4, especially 3;

where the R radicals are each independently selected from the group consisting of acyl radicals R'-C(=O)- and H, with the proviso that at least one R radical is not H; where the R' radicals are each independently selected from the group consisting of monovalent aliphatic, saturated or unsaturated hydrocarbon radicals having 3 to 39, preferably 7 to 21 and especially 9 to 17 carbon atoms.

**5.** Use according to at least one of Claims 1 to 4, **characterized in that** the at least one emulsifier has an HLB value of not less than 9, preferably of not less than 10, especially of not less than 11.

**6.** Use according to at least one of Claims 1 to 5, **characterized in that** the at least one emulsifier is selected from ethoxylated sorbitan fatty acid esters.

**7.** Use according to at least one of Claims 1 to 6, **characterized in that** the acyloxy radicals of the at least one sorbitan fatty acid ester or ethoxylated sorbitan fatty acid ester have 4 to 40, preferably 8 to 22 and especially 10 to 18 carbon atoms and/or **in that** the at least one sorbitan fatty acid ester or ethoxylated sorbitan fatty acid ester has 0 to 40, preferably 10 to 30 and especially 15 to 25 oxyethylene groups.

**8.** Use according to at least one of Claims 1 to 7, **characterized in that** the at least one polyglycerol ester is triglycerol trioleate and/or the at least one emulsifier is polyethylene glycol-20 sorbitan trioleate.

**9.** Use according to at least one of Claims 1 to 8, **characterized in that** the proportion by mass of the at least one polyglycerol ester based on the total mass of the composition is 60% to 100%, preferably 70% to 90%, especially 75% to 85%; and the proportion by mass of the at least one emulsifier based on the total mass of the composition is 0% to 40%, preferably 10% to 30%, especially 15% to 25%.

**10.** Use according to at least one of Claims 1 to 9, **characterized in that** the at least one active microbiological ingredient is selected from the group consisting of *Trichoderma harzianum, Bacillus amyloliquefaciens, Beauveria bassiana, Metarhizium rileyi, Metarhizium anisopliae, Clonostachys rosea, Aureobasidium pullulans, Coniothyrium minitans* and organs thereof, where the organs are preferably selected from the group consisting of spores, conidia, blastospores, chlamydospores, sclerotia and hyphal segments.

**11.** Use according to at least one of Claims 1 to 10, **characterized in that** the at least one active microbiological ingredient comprises spores, preferably fungal spores and/or bacterial spores, especially spores of *Trichoderma harzianum* and/or of *Bacillus amyloliquefaciens* and/or of *Beauveria bassiana* and/or of *Metarhizium rileyi* and/or of *Metarhizium anisopliae* and/or of *Clonostachys rosea* and/or of *Aureobasidium pullulans* and/or of *Coniothyrium minitans.*

**12.** Use according to at least one of Claims 1 to 11, **characterized in that** the at least one active microbiological ingredient comprises or consists of spores of *Trichoderma harzianum.*

**13.** Use according to at least one of Claims 1 to 12, **characterized in that** the $a_w$ value of the composition is less than 0.4, preferably less than 0.3, especially less than 0.25.

**14.** Use according to at least one of Claims 1 to 13 for increasing the storage stability of the active microbiological ingredient.

**15.** Method of increasing the storage stability of at least one active microbiological ingredient by using a composition comprising at least one polyglycerol ester and preferably at least one emulsifier, as carrier for at least one active microbiological ingredient, according to the provisions of at least one of Claims 1 to 13.

**16.** Composition comprising:

(a) a carrier comprising:

(a1) at least one polyglycerol ester and preferably
(a2) at least one emulsifier selected from the group consisting of sorbitan fatty acid esters and ethoxylated sorbitan fatty acid esters, and

(b) at least one active microbiological ingredient;

according to the provisions of at least one of Claims 1 to 13, **characterized in that** the $a_w$ value of the active ingredient composition is less than 0.4, preferably less than 0.3, especially less than 0.25.

**17.** Use of the composition according to Claim 16

i) for the treatment of plants; or
ii) for the treatment of seed; or
iii) for the treatment of soils; or
iv) as biostimulant; or
v) as probiotic food supplement and/or probiotic animal feed additive.

**18.** Composition according to Claim 16 for use as probiotic medicament.

## Revendications

**1.** Utilisation d'une composition comprenant au moins un ester de polyglycérol et préférablement au moins un émulsifiant, comme véhicule pour au moins un principe actif microbiologique, **caractérisée en ce que** l'au moins un émulsifiant est choisi dans le groupe constitué d'esters d'acide gras et de sorbitane et d'esters d'acide gras et de sorbitane éthoxylés, et **caractérisée en outre en ce que** l'au moins un principe actif microbiologique est choisi dans le groupe constitué de micro-organismes, d'organites de micro-organismes et de leurs mélanges

**2.** Utilisation selon la revendication 1, **caractérisée en ce que** l'au moins un ester de polyglycérol présente une valeur HLB inférieure ou égale à 8, préférablement inférieure ou égale à 7, en particulier inférieure ou égale à 6,5.

**3.** Utilisation selon la revendication 1 ou 2, **caractérisée en ce que** les radicaux acyloxy de l'au moins un ester de polyglycérol présentent 4 à 40, préférablement 8 à 22, en particulier 10 à 18 atomes de carbone.

**4.** Utilisation selon au moins l'une des revendications 1 à 3, **caractérisée en ce que** l'au moins un ester de polyglycérol est un composé de formule générale (I),

$M_aD_bT_c$ Formule (I) ;

comportant :

$M = [C_3H_5(OR)_2O_{1/2}]$ ;
$D = [C_3H_5(OR)_1O_{2/2}]$ ;
$T = [C_3H_5O_{3/2}]$ ;
a = 1 à 10, préférablement 2 à 3, en particulier 2 ;
b = 0 à 10, préférablement supérieur à 0 jusqu'à 5, en particulier 1 à 3 ;
c = 0 à 3, préférablement 0 à 1, en particulier 0 ;

à condition que :

a + b + c = 2 à 20, préférablement 2 à 4, en particulier 3 ;
dans laquelle les radicaux R sont choisis chacun indépendamment les uns des autres dans le groupe constitué de radicaux acyle R'-C(=O)- et H, à condition qu'au moins un radical R soit différent de H ;
les radicaux R' étant choisis chacun indépendamment les uns des autres dans le groupe constitué de radicaux hydrocarbonés aliphatiques monovalents, saturés ou
insaturés, ayant de 3 à 39, préférablement de 7 à 21, en particulier de 9 à 17 atomes de carbone.

**5.** Utilisation selon au moins l'une des revendications 1 à 4, **caractérisée en ce que** l'au moins un émulsifiant présente une valeur HLB supérieure ou égale à 9, préférablement supérieure ou égale à 10, en particulier supérieure ou égale à 11.

**6.** Utilisation selon au moins l'une des revendications 1 à 5, **caractérisée en ce que** l'au moins un émulsifiant est choisi parmi les esters d'acides gras et de sorbitane éthoxylés.

7. Utilisation selon au moins l'une des revendications 1 à 6, **caractérisée en ce que** les radicaux acyloxy de l'au moins un ester d'acide gras et de sorbitane ou ester d'acide gras et de sorbitane éthoxylé présentent 4 à 40, préférablement 8 à 22, en particulier 10 à 18 atomes de carbone et/ou **en ce que** l'au moins un ester d'acide gras et de sorbitane ou ester d'acide gras et de sorbitane éthoxylé présente 0 à 40, préférablement 10 à 30, en particulier 15 à 25 groupes oxyéthylène.

8. Utilisation selon au moins l'une des revendications 1 à 7, **caractérisée en ce que** l'au moins un ester de polyglycérol est le trioléate de triglycérol et/ou l'au moins un émulsifiant est le trioléate de polyéthylèneglycol-20-sorbitane.

9. Utilisation selon au moins l'une des revendications 1 à 8, **caractérisée en ce que** la fraction massique de l'au moins un ester de polyglycérol par rapport à la masse totale de la composition est de 60 % à 100 %, préférablement de 70 % à 90 %, en particulier de 75 % à 85 % ; et la fraction massique de l'au moins un émulsifiant par rapport à la masse totale de la composition est de 0 % à 40 %, préférablement de 10 % à 30 %, en particulier de 15 % à 25 %.

10. Utilisation selon au moins l'une des revendications 1 à 9, **caractérisée en ce que** l'au moins un principe actif microbiologique est choisi dans le groupe constitué de *Trichoderma harzianum, Bacillus amyloliquefaciens, Beauveria bassiana, Metarhizium rileyi, Metarhizium anisopliae, Clonostachys rosea, Aureobasidium pullulans, Coniothyrium minitans* et leurs organites, les organites étant préférablement choisis dans le groupe constitué de spores, de conidies, de blastospores, de chlamydospores, de sclérotes et de segments d'hyphes.

11. Utilisation selon au moins l'une des revendications 1 à 10, **caractérisée en ce que** l'au moins un principe actif microbiologique comprend des spores, préférablement des spores de champignons et/ou des spores de bactéries, en particulier des spores de *Trichoderma harzianum* et/ou de *Bacillus amyloliquefaciens* et/ou de *Beauveria bassiana* et/ou de *Metarhizium rileyi* et/ou de *Metarhizium anisopliae* et/ou de *Clonostachys rosea* et/ou de *Aureobasidium pullulans* et/ou de *Coniothyrium minitans.*

12. Utilisation selon au moins l'une des revendications 1 à 11, **caractérisée en ce que** l'au moins un principe actif microbiologique comprend ou est constitué de spores de *Trichoderma harzianum.*

13. Utilisation selon au moins l'une des revendications 1 à 12, **caractérisée en ce que** la valeur $a_w$ de la composition est inférieure à 0,4, préférablement inférieure à 0,3, en particulier inférieure à 0,25.

14. Utilisation selon au moins l'une des revendications 1 à 13 pour augmenter la stabilité au stockage du principe actif microbiologique.

15. Procédé pour augmenter la stabilité au stockage d'au moins un principe actif microbiologique par utilisation d'une composition comprenant au moins un ester de polyglycérol et préférablement au moins un émulsifiant, comme véhicule d'au moins un principe actif microbiologique, conformément aux spécifications selon au moins l'une des revendications 1 à 13.

16. Composition comprenant :

    (a) un véhicule comprenant :

        (a1) au moins un ester de polyglycérol et de préférence
        (a2) au moins un émulsifiant choisi dans le groupe constitué d'esters d'acides gras et de sorbitane et d'esters d'acides gras et de sorbitane éthoxylés, et

    (b) au moins un principe actif microbiologique ;

    conformément aux spécifications selon au moins l'une des revendications 1 à 13, **caractérisée en ce que** la valeur $a_w$ de la composition de principes actifs est inférieure à 0,4, préférablement inférieure à 0,3, en particulier inférieure à 0,25.

17. Utilisation de la composition selon la revendication 16

    i) pour le traitement de plantes ; ou
    ii) pour le traitement de semences ; ou

iii) pour le traitement de sols ; ou
iv) en tant que biostimulant ; ou
v) en tant que complément alimentaire probiotique et/ou additif probiotique pour aliments pour animaux.

**18.** Composition selon la revendication 16, destinée à être utilisée comme médicament probiotique.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2012163322 A1
- WO 2015069708 A1
- WO 2017210512 A1
- WO 2016055344 A1
- WO 2011026983 A1


### In der Beschreibung aufgeführte Nicht-Patentliteratur


- The Manual of Biocontrol Agents,. The British Crop Protection Council, 2001
- **W. C. GRIFFIN:**. Classification of surface active agents by HLB. *J. Soc. Cosmet. Chem*, 1949, vol. 1, 311-326
- The Manual of Biocontrol Agents. The British Crop Protection Council, 2001
- **H.D. BURGES**. Formulation of Microbial Biopesticides. Springer, 1998